# EUROPEAN PATENT APPLICATION

(11) **EP 4 115 920 A1**
(43) Date of publication of application: **11.01.2023**
(21) Application number: 21765167.8
(22) Date of filing: 04.03.2021
(51) Int. Cl.: A61M 1/16, A61M 60/109

(54) **EXTRACORPOREAL CIRCULATION DEVICE**

(30) Priority: 05.03.2020 JP 2020038054
(71) Applicant: TERUMO Kabushiki Kaisha, Tokyo 151-0072 (JP)
(72) Inventor: UTSUGIDA Tomoki, Ashigarakami-gun Kanagawa 259-0151 (JP); CHINO Naotaka, Ashigarakami-gun Kanagawa 259-0151 (JP); SUGIYAMA Yuichiro, Ashigarakami-gun Kanagawa 259-0151 (JP); YAMADA Makoto, Ashigarakami-gun Kanagawa 259-0151 (JP)
(74) Representative: Prüfer & Partner mbB Patentanwälte · Rechtsanwälte
(86) International application number: PCT/JP2021/008548
(87) International publication number: WO 2021/177423

(57) **Abstract**

[Problems to be solved] Provided is an extracorporeal circulator capable of judging a timing suitable for detachment with high accuracy.

[Means to solve the problems] An extracorporeal circulator 1 is an extracorporeal circulator for extracorporeal circulation of blood using a circulation path 1R. The extracorporeal circulator includes: a blood removal side catheter 5 configured to have a part inserted into a patient P and guide the blood taken from the patient P; a pump 3 configured to take the blood from the patient P and return the blood to the patient P; a blood transfer side catheter 6 provided downstream of the pump 3, and configured to have a part inserted into the patient P and guide the blood sent out from the pump 3 to the patient P; a pump rotation speed detection unit 36 configured to detect a rotation speed of the pump 3; and a control system 10 including at least one of an extracorporeal circulation management system configured to judge stability of a dynamic of the extracorporeal circulation and a cardiac function measurement system configured to judge stability of a heart function of the patient P.

## Description

### Technical Field

This disclosure relates to an extracorporeal circulator.

### Background Art

As one of professional resuscitation therapies for cardiogenic cardiac arrest patients, there is invasive cardiopulmonary resuscitation using an extracorporeal circulator as percutaneous cardiopulmonary support (PCPS). Such a resuscitation therapy may be called extracorporeal cardiopulmonary resuscitation (ECPR) or extracorporeal membrane oxygenation (ECMO).

In the invasive cardiopulmonary resuscitation using the extracorporeal circulator, one of necessary factors is to judge a timing suitable for detachment of the extracorporeal circulator. That is, when the timing of the detachment of the extracorporeal circulator is too early, a heart of the patient cannot withstand an operation, and the extracorporeal circulator may be necessary to be reattached, or the patient may die. On the other hand, when the timing of the detachment of the extracorporeal circulator is too late, complications such as bleeding may occur due to an anticoagulation therapy or consumption of blood plasma components. That is, there is an appropriate timing for the detachment of the extracorporeal circulator. Further, it is desirable that the timing suitable for the detachment of the extracorporeal circulator can be determined early.

A criterion for judging the timing suitable for the detachment of the extracorporeal circulator is, for example, stability of a heart function of a patient, in other words, a degree of recovery of a cardiac function of the patient. However, it is difficult to judge the recovery of the cardiac function of the patient with high accuracy.

That is, during the invasive cardiopulmonary resuscitation using the extracorporeal circulator, a pump of the extracorporeal circulator takes out blood from the patient, allows the blood to pass through an artificial lung, and returns the blood passed through the artificial lung to the patient. At this time, a direction of a flow of the blood returned into a body of the patient by the pump is opposite to a direction of a flow of blood flowing in the body of the patient. That is, the pump returns the blood passed through the artificial lung to the patient in a retrograde state. Therefore, even in a case where a flow rate sensor attached to a catheter is delivered to the vicinity of the heart of the patient, it is difficult to measure a cardiac output of the heart alone. Therefore, in this case, it is difficult to judge the recovery of the cardiac function of the patient with high accuracy.

In addition, in order to judge the recovery of the cardiac function, a facility that temporarily stops operation of the extracorporeal circulator and measures the cardiac output of the heart is also provided. However, in order to reduce a burden on the patient, it is desirable that the recovery of the cardiac function can be judged continuously rather than intermittently.

PTL 1 discloses a cardiac function evaluator that calculates a time differential of a blood flow rate measured by a blood flowmeter and evaluates a cardiac function based on a result of the calculation. The cardiac function evaluator described in PTL 1 converts the time differential of the blood flow rate into a time differential of a blood pressure. A relationship between the time differential of the blood flow rate and the time differential of the blood pressure is described in Fig. 2 of PTL 1. According to Fig. 2 described in PTL 1, an error of the time differential of the blood pressure is relatively large even though a cardiac function evaluation block including the blood flowmeter is provided in the vicinity of a heart of a patient. Therefore, as described above, even when the blood flowmeter is provided in the vicinity of the heart of the patient, it is difficult to judge the recovery of the cardiac function of the patient with high accuracy.

Another criterion for judging the timing suitable for the detachment of the extracorporeal circulator is, for example, stability of a dynamic of blood circulation in a circulation path of the extracorporeal circulator, that is, stability of a dynamic of extracorporeal circulation. However, the stability of the dynamic of the extracorporeal circulation is often judged based on experience and knowledge of a health care worker, and it is difficult to judge the stability of the dynamic of the extracorporeal circulation with high accuracy.

That is, one of factors causing the stability of the dynamic of the extracorporeal circulation to decrease is, for example, a blood removal failure due to a distal portion of a vein side cannula (blood removal side cannula) being suctioned against a blood vessel wall. When the blood removal failure occurs, the health care worker delivers, for example, a physiological salt solution to a vein or performs blood transfusion based on experience and knowledge. However, since supplement of a liquid such as a physiological salt solution and blood transfusion is not a method for eliminating a fundamental cause of the blood removal failure, the blood removal failure may occur again. Considering that the liquid such as a physiological salt solution may leak out of the blood vessel and cause edema, it is desirable to reduce an amount of the liquid such as a physiological salt solution to be supplemented or an amount of blood to be transfused.

As described above, for example, it is desirable that the timing suitable for the detachment of the extracorporeal circulator can be judged with high accuracy by judging the stability of the heart function of the patient with high accuracy or judging the stability of the dynamic of the extracorporeal circulation with high accuracy.

### Citation List

### Patent Literature

PTL 1: JP-A-2002-224066

### Summary of Invention

### Technical Problem

This disclosure is made to solve the above problems, and an object thereof is to provide an extracorporeal circulator capable of judging a timing suitable for detachment with high accuracy.

### Solution to Problem

The above problems are solved by an extracorporeal circulator for extracorporeal circulation of blood using a circulation path according to this disclosure. The extracorporeal circulator includes:
a blood removal side catheter configured to have a part inserted into a patient and guide the blood taken from the patient;
a pump configured to take the blood from the patient and return the blood to the patient;
a blood transfer side catheter provided downstream of the pump, and configured to have a part inserted into the patient and guide the blood sent out from the pump to the patient;
a pump rotation speed detection unit configured to detect a rotation speed of the pump; and
a control system including at least one of an extracorporeal circulation management system configured to judge stability of a dynamic of the extracorporeal circulation and a cardiac function measurement system configured to judge stability of a heart function of the patient.

According to the extracorporeal circulator of this disclosure, the control system includes at least one of the extracorporeal circulation management system and the cardiac function measurement system. The extracorporeal circulation management system judges the stability of the dynamic of the extracorporeal circulation. The cardiac function measurement system judges the stability of the heart function of the patient. As described above, the control system includes at least one of the extracorporeal circulation management system configured to judge the stability of the dynamic of the extracorporeal circulation as one of criteria for judging a timing suitable for detachment of the extracorporeal circulator, and the cardiac function measurement system configured to judge the stability of the heart function of the patient as one of the criteria for judging the timing suitable for the detachment of the extracorporeal circulator. Therefore, the control system of the extracorporeal circulator according to this disclosure can judge the timing suitable for the detachment of the extracorporeal circulator with high accuracy.

In the extracorporeal circulator according to this disclosure, the extracorporeal circulation management system may include
a pump blood transfer flow rate variation width determination unit configured to determine, based on a measurement result of a flow rate measurement unit provided in the circulation path, a pump blood transfer flow rate variation width indicating a variation width of a flow rate of the blood sent out from the pump at the rotation speed detected by the pump rotation speed detection unit,
a circulation stability determination unit configured to determine circulation stability indicating the stability of the dynamic of the extracorporeal circulation based on a reference blood transfer flow rate variation width indicating a standard variation width of the flow rate of the blood sent out from the pump and the pump blood transfer flow rate variation width determined by the pump blood transfer flow rate variation width determination unit, and
a circulation stability judgment unit configured to judge the stability of the dynamic of the extracorporeal circulation based on the circulation stability determined by the circulation stability determination unit.

According to the extracorporeal circulator of this disclosure, the extracorporeal circulation management system includes the pump blood transfer flow rate variation width determination unit, the circulation stability determination unit, and the circulation stability judgment unit. The pump blood transfer flow rate variation width determination unit determines the pump blood transfer flow rate variation width based on the measurement result of the flow rate measurement unit provided in the circulation path. The pump blood transfer flow rate variation width indicates the variation width of the flow rate of the blood sent out from the pump at the rotation speed of the pump detected by the pump rotation speed detection unit. The circulation stability determination unit determines the circulation stability based on the reference blood transfer flow rate variation width and the pump blood transfer flow rate variation width. The reference blood transfer flow rate variation width indicates the standard variation width of the flow rate of the blood sent out from the pump. The circulation stability indicates the stability of the dynamic of the extracorporeal circulation. Further, the circulation stability judgment unit judges the stability of the dynamic of the extracorporeal circulation based on the circulation stability determined by the circulation stability determination unit. As described above, the extracorporeal circulation management system determines the circulation stability based on the reference blood transfer flow rate variation width and the pump blood transfer flow rate variation width, and judges the stability of the dynamic of the extracorporeal circulation based on the circulation stability. Accordingly, the control system of the extracorporeal circulator according to this disclosure can judge the stability of the dynamic of the extracorporeal circulation with high accuracy, and can judge the timing suitable for the detachment of the extracorporeal circulator with high accuracy.

The control system of the extracorporeal circulator according to this disclosure can judge the stability of the dynamic of the extracorporeal circulation with high accuracy, and thus can capture a sign of a decrease in the stability of the dynamic of the extracorporeal circulation. Therefore, for example, the health care worker can adjust a position of a distal portion of the blood removal side catheter, supplement a liquid such as a physiological salt solution, transfuse blood, or the like at a more appropriate timing, and can prevent the decrease in the stability of the dynamic of the extracorporeal circulation. Accordingly, an amount of the liquid such as a physiological salt solution to be supplemented and an amount of the blood to be transfused can be reduced.

In the extracorporeal circulator according to this disclosure, the control system may further include a display processing unit configured to display, on a display unit, at least one of a graph indicating a relationship between the circulation stability and elapsed time and a numerical value indicating the circulation stability.

According to the extracorporeal circulator of this disclosure, the health care worker can easily check the circulation stability indicating the stability of the dynamic of the extracorporeal circulation by checking the display unit, and can visually grasp the timing suitable for the detachment of the extracorporeal circulator. By checking the display unit, for example, the health care worker can look back on interventions or procedures such as adjustment of the position of the distal portion of the blood removal side catheter, supplementation of the liquid such as a physiological salt solution, and blood transfusion.

In the extracorporeal circulator according to this disclosure, the cardiac function measurement system may include
an arterial pressure change width determination unit configured to determine, based on a measurement result of an arterial pressure measurement unit provided in the patient, an arterial pressure change width indicating a change width between a first arterial pressure of the blood flowing through an artery of the patient at a first rotation speed of the pump detected by the pump rotation speed detection unit and a second arterial pressure of the blood flowing through the artery of the patient at a second rotation speed of the pump detected by the pump rotation speed detection unit,
a heart function stability determination unit configured to determine heart function stability indicating the stability of the heart function of the patient based on a reference pressure change width indicating a standard change width between the first arterial pressure and the second arterial pressure and the arterial pressure change width determined by the arterial pressure change width determination unit, and
a heart function stability judgment unit configured to judge the stability of the heart function of the patient based on the heart function stability determined by the heart function stability determination unit.

According to the extracorporeal circulator of this disclosure, the cardiac function measurement system includes the arterial pressure change width determination unit, the heart function stability determination unit, and the heart function stability judgment unit. The arterial pressure change width determination unit determines the arterial pressure change width based on the measurement result of the arterial pressure measurement unit provided in the patient. The arterial pressure change width indicates the change width between the first arterial pressure and the second arterial pressure. The first arterial pressure is a pressure of the blood flowing through the artery of the patient at the first rotation speed of the pump detected by the pump rotation speed detection unit. The second arterial pressure is a pressure of the blood flowing through the artery of the patient at the second rotation speed of the pump detected by the pump rotation speed detection unit. The heart function stability determination unit determines the heart function stability based on the reference pressure change width and the arterial pressure change width determined by the arterial pressure change width determination unit. The reference pressure change width indicates the standard change width between the first arterial pressure and the second arterial pressure. The heart function stability indicates the stability of the heart function of the patient. Further, the heart function stability judgment unit judges the stability of the heart function of the patient based on the heart function stability determined by the heart function stability determination unit. As described above, the cardiac function measurement system determines the heart function stability based on the reference pressure change width and the arterial pressure change width, and judges the stability of the heart function of the patient based on the heart function stability. Accordingly, the control system of the extracorporeal circulator according to this disclosure can judge the stability of the heart function of the patient with high accuracy, and can judge the timing suitable for the detachment of the extracorporeal circulator with high accuracy.

Since the control system of the extracorporeal circulator according to this disclosure can judge the stability of the heart function of the patient with high accuracy, it is possible to prevent the timing of the detachment of the extracorporeal circulator from being too early or too late, to optimize attachment time of the extracorporeal circulator, and to reduce occurrence of complications.

In the extracorporeal circulator according to this disclosure, the control system may further include a display processing unit configured to display, on a display unit, at least one of a graph indicating a relationship between the heart function stability and elapsed time and a numerical value indicating the heart function stability.

According to the extracorporeal circulator of this disclosure, the health care worker can easily check the heart function stability indicating the stability of the heart function of the patient by checking the display unit, and can visually grasp the timing suitable for the detachment of the extracorporeal circulator.

In the extracorporeal circulator according to this disclosure,
the control system may include both the extracorporeal circulation management system and the cardiac function measurement system, and may include a comprehensive judgment system configured to judge stability of the entire extracorporeal circulator,
the extracorporeal circulation management system may include
   a pump blood transfer flow rate variation width determination unit configured to determine, based on a measurement result of a flow rate measurement unit provided in the circulation path, a pump blood transfer flow rate variation width indicating a variation width of a flow rate of the blood sent out from the pump at the rotation speed detected by the pump rotation speed detection unit, and
   a circulation stability determination unit configured to determine circulation stability indicating the stability of the dynamic of the extracorporeal circulation based on a reference blood transfer flow rate variation width indicating a standard variation width of the flow rate of the blood sent out from the pump and the pump blood transfer flow rate variation width determined by the pump blood transfer flow rate variation width determination unit,
the cardiac function measurement system may include
   an arterial pressure change width determination unit configured to determine, based on a measurement result of an arterial pressure measurement unit provided in the patient, an arterial pressure change width indicating a change width between a first arterial pressure of the blood flowing through an artery of the patient at a first rotation speed of the pump detected by the pump rotation speed detection unit and a second arterial pressure of the blood flowing through the artery of the patient at a second rotation speed of the pump detected by the pump rotation speed detection unit, and
   a heart function stability determination unit configured to determine heart function stability indicating the stability of the heart function of the patient based on a reference pressure change width indicating a standard change width between the first arterial pressure and the second arterial pressure and the arterial pressure change width determined by the arterial pressure change width determination unit, and
the comprehensive judgment system may include
   a comprehensive stability determination unit configured to determine comprehensive stability indicating comprehensive stability of the extracorporeal circulator based on the circulation stability determined by the circulation stability determination unit and the heart function stability determined by the heart function stability determination unit, and
   a comprehensive stability judgment unit configured to judge the comprehensive stability of the extracorporeal circulator based on the comprehensive stability determined by the comprehensive stability determination unit.

According to the extracorporeal circulator of this disclosure, the control system includes both the extracorporeal circulation management system and the cardiac function measurement system, and includes the comprehensive judgment system configured to judge the stability of the entire extracorporeal circulator.

The extracorporeal circulation management system includes the pump blood transfer flow rate variation width determination unit and the circulation stability determination unit. The pump blood transfer flow rate variation width determination unit determines the pump blood transfer flow rate variation width based on the measurement result of the flow rate measurement unit provided in the circulation path. The pump blood transfer flow rate variation width indicates the variation width of the flow rate of the blood sent out from the pump at the rotation speed of the pump detected by the pump rotation speed detection unit. The circulation stability determination unit determines the circulation stability based on the reference blood transfer flow rate variation width and the pump blood transfer flow rate variation width. The reference blood transfer flow rate variation width indicates the standard variation width of the flow rate of the blood sent out from the pump. The circulation stability indicates the stability of the dynamic of the extracorporeal circulation.

The cardiac function measurement system includes the arterial pressure change width determination unit and the heart function stability determination unit. The arterial pressure change width determination unit determines the arterial pressure change width based on the measurement result of the arterial pressure measurement unit provided in the patient. The arterial pressure change width indicates the change width between the first arterial pressure and the second arterial pressure. The first arterial pressure is a pressure of the blood flowing through the artery of the patient at the first rotation speed of the pump detected by the pump rotation speed detection unit. The second arterial pressure is a pressure of the blood flowing through the artery of the patient at the second rotation speed of the pump detected by the pump rotation speed detection unit. The heart function stability determination unit determines the heart function stability based on the reference pressure change width and the arterial pressure change width determined by the arterial pressure change width determination unit. The reference pressure change width indicates the standard change width between the first arterial pressure and the second arterial pressure. The heart function stability indicates the stability of the heart function of the patient.

The comprehensive judgment system includes the comprehensive stability determination unit and the comprehensive stability judgment unit. The comprehensive stability determination unit determines the comprehensive stability based on the circulation stability determined by the circulation stability determination unit and the heart function stability determined by the heart function stability determination unit. The comprehensive stability indicates the comprehensive stability of the extracorporeal circulator. Further, the comprehensive stability judgment unit judges the comprehensive stability of the extracorporeal circulator based on the comprehensive stability determined by the comprehensive stability determination unit. Accordingly, the control system of the extracorporeal circulator according to this disclosure can judge both the stability of the dynamic of the extracorporeal circulation and the stability of the heart function of the patient with high accuracy, and can judge the timing suitable for the detachment of the extracorporeal circulator with higher accuracy.

In the extracorporeal circulator according to this disclosure, the control system may further include a display processing unit configured to display, on a display unit, at least one of a graph indicating a relationship between the circulation stability and elapsed time, a numerical value indicating the circulation stability, a graph indicating a relationship between the heart function stability and elapsed time, a numerical value indicating the heart function stability, a graph indicating a relationship between the comprehensive stability and elapsed time, and a numerical value indicating the comprehensive stability.

According to the extracorporeal circulator of this disclosure, by checking the display unit, the health care worker can easily check the circulation stability indicating the stability of the dynamic of the extracorporeal circulation, the heart function stability indicating the stability of the heart function of the patient, and the comprehensive stability indicating the comprehensive stability of the extracorporeal circulator, and can more reliably and visually grasp the timing suitable for the detachment of the extracorporeal circulator.

### Advantageous Effect of Invention

According to this disclosure, an extracorporeal circulator capable of judging a timing suitable for detachment with high accuracy can be provided.

### Brief Description of Drawings

[Fig. 1] Fig. 1 is a schematic diagram showing an extracorporeal circulator according to an embodiment of this disclosure.
[Fig. 2] Fig. 2 is a schematic diagram illustrating a flow of blood returned from the extracorporeal circulator to a patient and a flow of blood sent out from a heart of the patient.
[Fig. 3] Fig. 3 is a block diagram illustrating a control unit and a flow rate measurement unit according to the present embodiment.
[Fig. 4] Fig. 4 is a block diagram illustrating a configuration of a main part of a control system according to the present embodiment.
[Fig. 5] Fig. 5 is a block diagram illustrating the configuration of the main part of the control system according to the present embodiment.
[Fig. 6] Fig. 6 shows graphs illustrating circulation stability.
[Fig. 7] Fig. 7 shows graphs illustrating heart function stability.
[Fig. 8] Fig. 8 is a flowchart illustrating a first specific example of operation of the extracorporeal circulator according to the present embodiment.
[Fig. 9] Fig. 9 is a flowchart illustrating the first specific example of the operation of the extracorporeal circulator according to the present embodiment.
[Fig. 10] Fig. 10 is a graph illustrating an example of a relationship between a rotation speed of a centrifugal pump and a blood transfer flow rate of the centrifugal pump.
[Fig. 11] Fig. 11 is a graph illustrating an example of a relationship between the circulation stability and elapsed time.
[Fig. 12] Fig. 12 is a schematic diagram illustrating an example of a screen on which both a graph indicating the relationship between the circulation stability and the elapsed time and a numerical value indicating the circulation stability are displayed on a display unit.
[Fig. 13] Fig. 13 is a flowchart illustrating a second specific example of the operation of the extracorporeal circulator according to the present embodiment.
[Fig. 14] Fig. 14 is a flowchart illustrating the second specific example of the operation of the extracorporeal circulator according to the present embodiment.
[Fig. 15] Fig. 15 is a graph illustrating an example of a relationship between the rotation speed of the centrifugal pump and an average arterial pressure.
[Fig. 16] Fig. 16 is a graph illustrating an example of a relationship between the heart function stability and the elapsed time.
[Fig. 17] Fig. 17 is a schematic diagram illustrating an example of a screen on which both a graph indicating the relationship between the heart function stability and the elapsed time and a numerical value indicating the heart function stability are displayed on the display unit.
[Fig. 18] Fig. 18 is a flowchart illustrating a third specific example of the operation of the extracorporeal circulator according to the present embodiment.
[Fig. 19] Fig. 19 is a flowchart illustrating the third specific example of the operation of the extracorporeal circulator according to the present embodiment.
[Fig. 20] Fig. 20 is a timing chart illustrating operation of the specific example.
[Fig. 21] Fig. 21 is a timing chart illustrating operation of a comparative example.

### Description of Embodiments

Hereinafter, a preferred embodiment of this disclosure will be described in detail with reference to the drawings.

The embodiment to be described below is a preferred specific example of this disclosure, and thus various technically preferable limitations are applied thereto. However, unless there is any particular mention which limits this disclosure in the description below, the range of this disclosure is not limited to these aspects. In the drawings, the same components are denoted by the same reference numerals, and a detailed description thereof will be omitted appropriately.

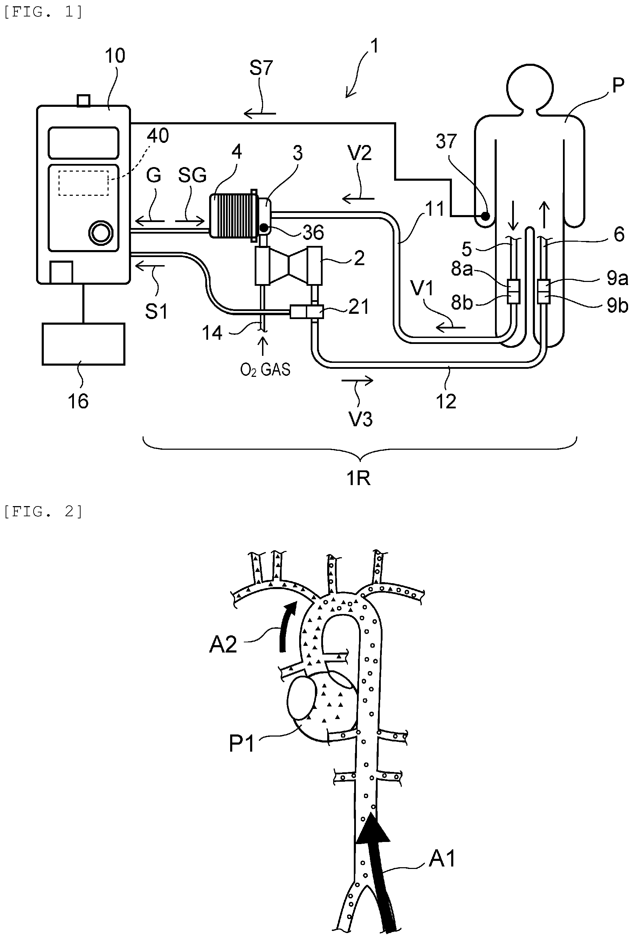

Fig. 1 is a schematic diagram showing an extracorporeal circulator according to the embodiment of this disclosure.

"Extracorporeal circulation" performed by an extracorporeal circulator 1 shown in Fig. 1 includes an "extracorporeal circulation operation" and an "auxiliary circulation operation". The extracorporeal circulator 1 can perform both the "extracorporeal circulation operation" and the "auxiliary circulation operation".

The "extracorporeal circulation operation" means that when blood circulation in a heart is temporarily stopped due to, for example, cardiac surgery, the extracorporeal circulator 1 performs a blood circulation operation and a gas exchange operation (oxygen addition and/or carbon dioxide removal) for the blood.

The "auxiliary circulation operation" means that in a case where a heart of a patient P to which the extracorporeal circulator 1 is applied cannot perform a sufficient function or in a state where a lung cannot perform gas exchange sufficiently, the extracorporeal circulator 1 also performs the blood circulation operation and the gas exchange operation for the blood. In the description of the present embodiment, the "auxiliary circulation operation" is mainly taken as an example.

For example, the extracorporeal circulator 1 is applied to a case where the heart of the patient P does not operate normally, a case where the heart of the patient P operates normally but the lung does not operate normally, and the like. Accordingly, the extracorporeal circulator 1 shown in Fig. 1 is used, for example, in a case of performing a cardiac surgery on the patient P, in subsequent treatment in ICU, and the like. The extracorporeal circulator 1 shown in Fig. 1 can perform artificial lung extracorporeal blood circulation in which a pump of the extracorporeal circulator 1 is operated to remove blood from a vein of the patient, an artificial lung performs gas exchange in the blood to oxygenate the blood, and then the oxygenated blood is returned to an artery or the vein of the patient. As described above, the extracorporeal circulator 1 is a device that operates in place of the heart and the lung.

As shown in Fig. 1, the extracorporeal circulator 1 includes a circulation path 1R for circulating the blood. The circulation path 1R includes an artificial lung 2, a centrifugal pump 3, a drive motor 4 that drives the centrifugal pump 3, a blood removal side catheter (vein side catheter) 5, a blood transfer side catheter (artery side catheter) 6, and a control system 10. The centrifugal pump 3 according to the present embodiment is an example of the "pump" of this disclosure. The control system 10 includes a control unit 40 and is provided as a controller of the extracorporeal circulator 1. The centrifugal pump 3 is also referred to as a blood pump or the like, and may be a pump other than a centrifugal pump.

The blood removal side catheter 5 is also called a vein side cannula (blood removal side cannula) or the like, and is inserted from a femoral vein. A distal end of the blood removal side catheter 5 indwells in a right atrium. A proximal portion of the blood removal side catheter 5 is disposed outside the femoral vein. The blood removal side catheter 5 is connected to a blood removal tube 11 in a liquid-tight manner via a connector 8a provided at the proximal portion of the blood removal side catheter 5 and a connector 8b provided at a distal end of the blood removal tube (also referred to as a blood removal line) 11, is connected to the centrifugal pump 3 via the blood removal tube 11, and guides the blood taken from the patient P to the centrifugal pump 3 via the blood removal tube 11. The blood removal tube 11 is a duct that connects the blood removal side catheter 5 and the centrifugal pump 3, and is a duct that guides the blood taken from the patient P to the centrifugal pump 3 via the blood removal side catheter 5. The connectors 8a and 8b are formed such that a pressure loss of a blood flow does not occur inside the blood removal side catheter 5 or the blood removal tube 11.

The blood transfer side catheter 6 is also called an artery side cannula (blood transfer side cannula) or the like, and is inserted from a femoral artery. A distal end of the blood transfer side catheter 6 indwells in the femoral artery. A proximal portion of the blood transfer side catheter 6 is disposed outside the femoral artery. The blood transfer side catheter 6 is connected to a blood transfer tube 12 in a liquid-tight manner via a connector 9a provided at the proximal portion of the blood transfer side catheter 6 and a connector 9b provided at a distal end of the blood transfer tube (also referred to as a blood transfer line) 12, is connected to the artificial lung 2 via the blood transfer tube 12, and guides the blood passed through the artificial lung 2 to the patient P via the blood transfer tube 12. The blood transfer tube 12 is a duct that connects the artificial lung 2 and the blood transfer side catheter 6, and is a duct that guides the blood passed through the artificial lung 2 to the patient P. The connectors 9a and 9b are formed such that the pressure loss of the blood flow does not occur in the blood transfer side catheter 6 or the blood transfer tube 12.

The drive motor 4 controls driving of the centrifugal pump 3 based on a command SG from the control system 10. The centrifugal pump 3 is provided downstream of the blood removal side catheter 5, and is driven by receiving a driving force transmitted from the drive motor 4. The centrifugal pump 3 takes out the blood from the patient P through the blood removal side catheter 5 and the blood removal tube 11, sends out the blood to the artificial lung 2, and then returns the blood to the patient P through the blood transfer tube 12 and the blood transfer side catheter 6. At least one of the centrifugal pump 3 and the drive motor 4 includes a pump rotation speed detection unit 36. The pump rotation speed detection unit 36 detects a rotation speed of the centrifugal pump 3 and transmits a signal G related to the rotation speed of the centrifugal pump 3 to the control system 10.

The artificial lung 2 is provided downstream of the centrifugal pump 3. Specifically, the artificial lung 2 is disposed between the centrifugal pump 3 and the blood transfer tube 12. The artificial lung 2 performs a gas exchange operation (oxygen addition and/or carbon dioxide removal) for the blood. The artificial lung 2 is, for example, a membrane-type artificial lung, and is particularly preferably a hollow fiber membrane-type artificial lung. Oxygen gas is supplied to the artificial lung 2 through an oxygen supply tube 14.

As the blood removal tube 11 and the blood transfer tube 12, for example, a duct made of a highly transparent, elastically deformable, flexible synthetic resin such as a vinyl chloride resin or silicone rubber is used. The blood, which is a liquid, flows in a V1 direction and a V2 direction in the blood removal tube 11, and flows in a V3 direction in the blood transfer tube 12.

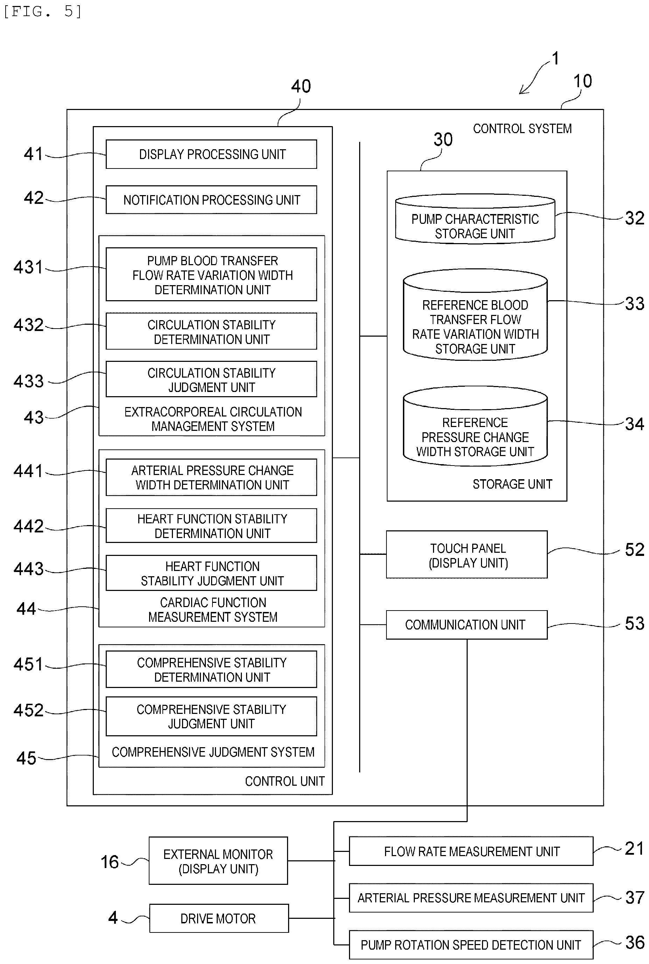

The control system 10 acquires various kinds of information, performs calculation, generates a control signal for controlling operations of devices such as the drive motor 4 and an external monitor 16, and transmits the control signal to each device. In other words, the control system 10 manages the extracorporeal circulator 1. Details of the control system 10 will be described later. In addition, the control system 10 may include a touch panel 52 (see Fig. 5) as an input unit for inputting various types of information and as a display unit that displays various types of information. That is, the "display unit" of this disclosure may be the external monitor 16 provided separately from the control system 10, or may be the touch panel 52 in the control system 10. The touch panel 52 can detect contact of a finger of an operator or the like.

The extracorporeal circulator 1 according to the present embodiment further includes a flow rate measurement unit 21, an arterial pressure measurement unit 37, and the external monitor (display unit) 16. The external monitor 16 according to the present embodiment is an example of the "display unit" of this disclosure. In the following description, a case where the "display unit" of this disclosure is the external monitor 16 will be described as an example.

The flow rate measurement unit 21 is provided in the circulation path 1R. In the extracorporeal circulator 1 according to the present embodiment, the flow rate measurement unit 21 is provided in the circulation path 1R between the artificial lung 2 and the blood transfer side catheter 6. Specifically, the flow rate measurement unit 21 is provided in the blood transfer tube 12. By providing the flow rate measurement unit 21 in the blood transfer tube 12, the flow rate measurement unit 21 can measure a flow rate of the blood immediately before being returned to the patient P, and can measure the flow rate of the blood flowing through the circulation path 1R at a position relatively close to the patient P. However, an installation position of the flow rate measurement unit 21 is not limited to the blood transfer tube 12, and may be any position in the circulation path 1R. In the following description, a case where the flow rate measurement unit 21 is provided in the blood transfer tube 12 will be described as an example.

The flow rate measurement unit 21 is, for example, a non-invasive flow rate sensor, is provided at an appropriate position outside the blood transfer tube 12, and detects the flow rate of the blood flowing inside the circulation path 1R. The flow rate measurement unit 21 shown in Fig. 1 measures the flow rate of the blood sent out from the centrifugal pump 3. The flow rate measurement unit 21 transmits a signal S1 related to the flow rate of the blood flowing inside the circulation path 1R to the control system 10. In the present embodiment, the flow rate measurement unit 21 transmits the signal S1 related to the flow rate of the blood sent out from the centrifugal pump 3 to the control system 10. An example of the flow rate measurement unit 21 is an ultrasonic flowmeter. As the ultrasonic flowmeter, for example, an ultrasonic transit time difference type flowmeter is used. However, the flow rate measurement unit 21 is not limited to the ultrasonic flowmeter.

The arterial pressure measurement unit 37 is provided, for example, at a position corresponding to a radial artery of a wrist of the patient P, and measures a pressure (arterial pressure) of the blood flowing through the artery of the patient P. The arterial pressure measurement unit 37 may measure the arterial pressure of the patient P by an invasive arterial pressure measurement method, or may measure the arterial pressure of the patient P by a non-invasive arterial measurement method. The arterial pressure measurement unit 37 is, for example, a pressure sensor, and transmits a signal S7 related to the pressure (arterial pressure) of the blood flowing through the artery of the patient P to the control system 10. A specific example of the arterial pressure measurement unit 37 using the non-invasive arterial measurement method is, for example, a tonometry pressure sensor that measures a pressure pulse wave by directly pressing a pressure sensor against a skin and calculates a blood pressure from the pressure pulse wave.

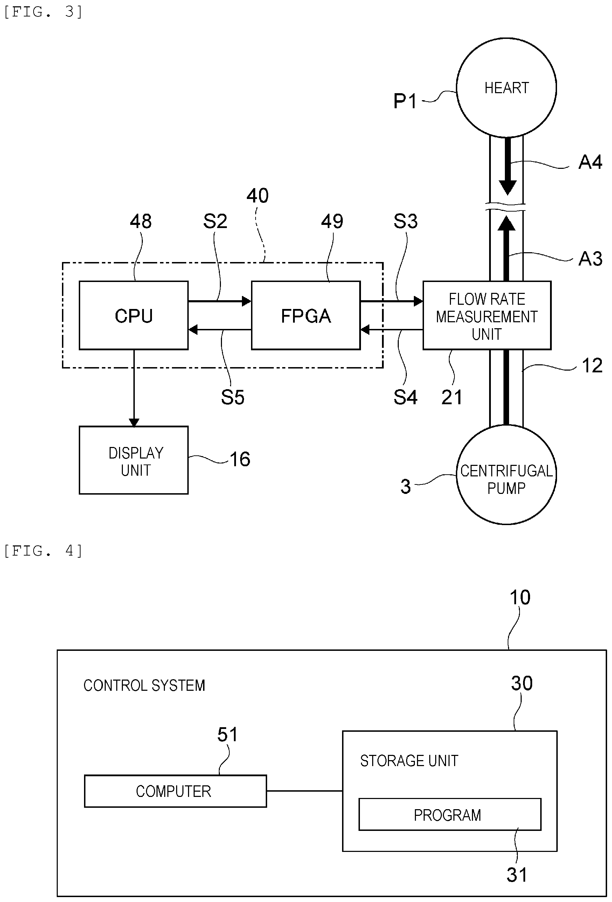

Fig. 2 is a schematic diagram illustrating a flow of the blood returned from the extracorporeal circulator to the patient and a flow of the blood sent out from the heart of the patient. Fig. 3 is a block diagram illustrating the control unit and the flow rate measurement unit according to the present embodiment. In the block diagram shown in Fig. 3, the artificial lung is omitted for convenience of illustration.

As indicated by an arrow A1 shown in Fig. 2 and an arrow A3 shown in Fig. 3, the blood returned from the centrifugal pump 3 to the patient P via the blood transfer tube 12 and the blood transfer side catheter 6 flows, for example, toward a heart P1 of the patient P through the artery. On the other hand, as indicated by an arrow A2 shown in Fig. 2 and an arrow A4 shown in Fig. 3, the blood sent out from the heart P1 of the patient P and oxygenated in the lung of the patient P passes through the artery and flows toward a peripheral blood vessel of a head and a peripheral blood vessel of a lower limb.

As described above, a direction of the flow of the blood returned into a body of the patient P by the centrifugal pump 3 is opposite to a direction of a flow of the blood flowing through the body of the patient P. That is, the centrifugal pump 3 returns the blood passed through the artificial lung 2 to the patient P in a retrograde state.

As shown in Fig. 3, the control unit 40 of the control system 10 includes a central processing unit (CPU) 48 and a field programmable gate array (FPGA) 49. For example, the CPU 48 transmits a signal S2 requesting measurement of the flow rate of the blood sent out from the centrifugal pump 3 to the FPGA 49 every 50 milliseconds (ms). The FPGA 49 receives the signal S2 transmitted from the CPU 48, and transmits a signal S3 requesting the measurement of the flow rate of the blood sent out from the centrifugal pump 3 to the flow rate measurement unit 21 every 50 ms. The flow rate measurement unit 21 receives the signal S3 transmitted from the FPGA 49, measures the flow rate of the blood sent out from the centrifugal pump 3 every 50 ms, and transmits a signal S4 related to the measured flow rate of the blood to the FPGA 49. The FPGA 49 receives the signal S4 transmitted from the flow rate measurement unit 21, and transmits a signal S5 related to an average value of the flow rate in the latest one second to the CPU 48.

In the present embodiment, since the flow rate measurement unit 21 measures the flow rate of the blood every 50 ms and transmits the signal S4 related to the measured flow rate of the blood to the FPGA 49, 20 pieces (1 s/50 ms) of measurement data are present per second. Therefore, the FPGA 49 calculates an average value of 20 pieces of measurement data as the average value of the flow rate in the latest one second, and transmits the signal S5 related to the average value of the flow rates to the CPU 48. The CPU 48 receives the signal S5 transmitted from the FPGA 49, and executes control to display the flow rate of the blood sent out from the centrifugal pump 3 on the external monitor (display unit) 16 as a pump blood transfer flow rate. In the present embodiment, the pump blood transfer flow rate is the average value of the flow rate in the latest one second, that is, the average value of 20 pieces of measurement data.

A period in which the flow rate measurement unit 21 measures the flow rate of the blood is not limited to 50 ms. Predetermined time for the CPU 48 to calculate the average value of the flow rate of the blood is not limited to one second.

Fig. 4 is a block diagram illustrating a configuration of a main part of the control system according to the present embodiment.

The control system 10 according to the present embodiment includes a computer 51 and a storage unit 30. The computer 51 includes the control unit 40 (see Figs. 1 and 5), reads a program 31 stored in the storage unit 30, and executes various calculations and processes. The storage unit 30 stores the program 31 (control program) executed by the computer 51. The program 31 according to the present embodiment is an example of the "control program" of this disclosure. Examples of the storage unit 30 include a hard disk drive (HDD). The program 31 is not limited to being stored in the storage unit 30, and may be stored and distributed in advance in a computer-readable storage medium, or may be downloaded to the control system 10 via a network. The storage unit 30 may be an external storage device connected to the computer 51.

Next, the configuration of the main part of the control system 10 according to the present embodiment will be further described with reference to the drawings.

Fig. 5 is a block diagram illustrating the configuration of the main part of the control system according to the present embodiment.

The control system 10 according to the present embodiment includes the control unit 40, the storage unit 30, the touch panel 52, and a communication unit 53. The control unit 40 reads the program 31 (see Fig. 4) stored in the storage unit 30 and executes various calculations and processes. The control unit 40 includes a display processing unit 41, a notification processing unit 42, an extracorporeal circulation management system 43, a cardiac function measurement system 44, and a comprehensive judgment system 45. The display processing unit 41, the notification processing unit 42, the extracorporeal circulation management system 43, the cardiac function measurement system 44, and the comprehensive judgment system 45 are implemented by executing the program 31 stored in the storage unit 30 by the computer 51. The display processing unit 41, the notification processing unit 42, the extracorporeal circulation management system 43, the cardiac function measurement system 44, and the comprehensive judgment system 45 may be implemented by hardware or a combination of hardware and software. The storage unit 30 stores the program 31 described above with reference to Fig. 4, and includes a pump characteristic storage unit 32, a reference blood transfer flow rate variation width storage unit 33, and a reference pressure change width storage unit 34.

The display processing unit 41 displays, on the external monitor 16, at least one of a graph indicating a relationship between circulation stability and elapsed time, a numerical value indicating the circulation stability, a graph indicating a relationship between heart function stability and elapsed time, a numerical value indicating the heart function stability, a graph indicating a relationship between comprehensive stability and elapsed time, and a numerical value indicating the comprehensive stability. The "circulation stability" indicates stability of a dynamic of blood circulation in the circulation path 1R of the extracorporeal circulator 1, that is, stability of a dynamic of extracorporeal circulation. The "heart function stability" indicates a degree of recovery of a cardiac function of the patient P, that is, stability of a heart function of the patient P. The "comprehensive stability" indicates comprehensive stability of the extracorporeal circulator 1 including the circulation stability, the heart function stability, and the like. Details of the circulation stability, the heart function stability, and the comprehensive stability will be described later.

The notification processing unit 42 performs notification on the external monitor 16 when a predetermined condition is satisfied. For example, when the circulation stability decreases or is low, the notification processing unit 42 displays on the external monitor 16 that the circulation stability decreases or is low. For example, when the heart function stability decreases or is low, the notification processing unit 42 displays on the external monitor 16 that the heart function stability decreases or is low. For example, when the comprehensive stability decreases or is low, the notification processing unit 42 displays on the external monitor 16 that the comprehensive stability decreases or is low. For example, when the predetermined condition is satisfied, the notification processing unit 42 displays on the external monitor 16 that a current timing is suitable for detachment of the extracorporeal circulator 1, or displays on the external monitor 16 that the current timing is late for the detachment of the extracorporeal circulator 1. The notification processing unit 42 may execute a notification method by, for example, generating light or sound. In the specification of the present application, "detachment of the extracorporeal circulator 1" includes both a case where the extracorporeal circulator 1 is detached from the patient P by disconnecting the connector 8a and the connector 8b from each other and disconnecting the connector 9a and the connector 9b from each other, and a case where the extracorporeal circulator 1 is detached from the patient P by removing the blood removal side catheter 5 and the blood transfer side catheter 6 from the patient P.

The extracorporeal circulation management system 43 includes a pump blood transfer flow rate variation width determination unit 431, a circulation stability determination unit 432, and a circulation stability judgment unit 433. The pump blood transfer flow rate variation width determination unit 431 determines a pump blood transfer flow rate variation width based on a measurement result of the flow rate measurement unit 21. The pump blood transfer flow rate variation width indicates a variation width of the flow rate of the blood sent out from the centrifugal pump 3 at the rotation speed of the centrifugal pump 3 detected by the pump rotation speed detection unit 36. For example, the pump blood transfer flow rate variation width determination unit 431 determines the pump blood transfer flow rate variation width at the rotation speed of the centrifugal pump 3 based on the measurement data of the flow rate measurement unit 21 in the latest predetermined time. Alternatively, the pump blood transfer flow rate variation width determination unit 431 may determine the pump blood transfer flow rate variation width at the rotation speed of the centrifugal pump 3 based on, for example, a table stored in the pump characteristic storage unit 32. Alternatively, the pump blood transfer flow rate variation width determination unit 431 may determine the pump blood transfer flow rate variation width at the rotation speed of the centrifugal pump 3 based on, for example, a calculation formula stored in the pump characteristic storage unit 32 and at least one of the rotation speed of the centrifugal pump 3 detected by the pump rotation speed detection unit 36 and the measurement data of the flow rate measurement unit 21. The table and the calculation formula described above may be stored in another portion of the storage unit 30 instead of the pump characteristic storage unit 32.

The circulation stability determination unit 432 determines the circulation stability based on a reference blood transfer flow rate variation width and the pump blood transfer flow rate variation width determined by the pump blood transfer flow rate variation width determination unit 431. The "reference blood transfer flow rate variation width" indicates a standard variation width of the flow rate of the blood sent out from the centrifugal pump 3. For example, the circulation stability determination unit 432 reads the reference blood transfer flow rate variation width stored in the reference blood transfer flow rate variation width storage unit 33. Alternatively, the circulation stability determination unit 432 may determine the reference blood transfer flow rate variation width based on a pump characteristic indicating a relationship between a flow rate of the centrifugal pump 3 and a discharge pressure (that is, a pump head) of the centrifugal pump 3. The pump characteristic is stored in, for example, the pump characteristic storage unit 32. The circulation stability is as described above.

The circulation stability judgment unit 433 judges the stability of the dynamic of the extracorporeal circulation, that is, the stability of the dynamic of the blood circulation in the circulation path 1R of the extracorporeal circulator 1, based on the circulation stability determined by the circulation stability determination unit 432. For example, when the circulation stability determined by the circulation stability determination unit 432 is less than a predetermined threshold value, the circulation stability judgment unit 433 judges that the dynamic of the extracorporeal circulation is not stable. On the other hand, when the circulation stability determined by the circulation stability determination unit 432 is equal to or greater than the predetermined threshold value, the circulation stability judgment unit 433 judges that the dynamic of the extracorporeal circulation is stable. A judgment method of the circulation stability judgment unit 433 is not limited thereto.

The cardiac function measurement system 44 includes an arterial pressure change width determination unit 441, a heart function stability determination unit 442, and a heart function stability judgment unit 443. The arterial pressure change width determination unit 441 determines an arterial pressure change width based on a measurement result of the arterial pressure measurement unit 37. The arterial pressure change width indicates a change width (for example, a difference) between arterial pressures of the blood flowing through the artery of the patient P at different rotation speeds of the centrifugal pump 3 detected by the pump rotation speed detection unit 36. For example, the arterial pressure change width determination unit 441 calculates, based on measurement data of the arterial pressure measurement unit 37 in the latest predetermined time, a first arterial pressure of the blood flowing through the artery of the patient P at a first rotation speed (previous rotation speed) of the centrifugal pump 3 detected by the pump rotation speed detection unit 36 and a second arterial pressure of the blood flowing through the artery of the patient P at a second rotation speed (latest rotation speed) of the centrifugal pump 3 detected by the pump rotation speed detection unit 36. Further, the arterial pressure change width determination unit 441 determines the arterial pressure change width indicating a change width between the first arterial pressure and the second arterial pressure.

The "arterial pressure" in the specification of the present application may be an average value (average arterial pressure) of measurement data of the arterial pressure measurement unit 37 in predetermined time, a systolic arterial pressure (highest arterial pressure), or a diastolic arterial pressure (lowest arterial pressure). Alternatively, the "arterial pressure" in the specification of the present application may be a difference between the highest arterial pressure and the lowest arterial pressure, or may be amplitude of each of the highest arterial pressure and the lowest arterial pressure.

The heart function stability determination unit 442 determines the heart function stability based on a reference pressure change width and the arterial pressure change width determined by the arterial pressure change width determination unit 441. The "reference pressure change width" indicates a standard change width (for example, a difference) between the first arterial pressure at the first rotation speed (previous rotation speed) of the centrifugal pump 3 and the second arterial pressure at the second rotation speed (latest rotation speed) of the centrifugal pump 3. For example, the heart function stability determination unit 442 reads the reference pressure change width stored in the reference pressure change width storage unit 34. The reference pressure change width may be stored in the reference pressure change width storage unit 34 as a table set according to information (for example, height, weight, age, sex, and the like) on the patient P, for example. The heart function stability is as described above.

The heart function stability judgment unit 443 judges the stability of the heart function of the patient P, that is, the degree of the recovery of the cardiac function of the patient P, based on the heart function stability determined by the heart function stability determination unit 442. For example, when the heart function stability determined by the heart function stability determination unit 442 is less than a predetermined threshold value, the heart function stability judgment unit 443 judges that the heart function of the patient P is not stable, that is, the degree of the recovery of the cardiac function of the patient P is low. On the other hand, when the heart function stability determined by the heart function stability determination unit 442 is equal to or higher than the predetermined threshold value, the heart function stability judgment unit 443 judges that the heart function of the patient P is stable, that is, the degree of the recovery of the cardiac function of the patient P is high. A judgment method of the heart function stability judgment unit 443 is not limited thereto.

The comprehensive judgment system 45 includes a comprehensive stability determination unit 451 and a comprehensive stability judgment unit 452. The comprehensive stability determination unit 451 determines the comprehensive stability based on the circulation stability determined by the circulation stability determination unit 432 and the heart function stability determined by the heart function stability determination unit 442. The comprehensive stability is as described above.

The comprehensive stability judgment unit 452 judges the comprehensive stability of the extracorporeal circulator 1 based on the comprehensive stability determined by the comprehensive stability determination unit 451. For example, when the comprehensive stability determined by the comprehensive stability determination unit 451 is less than a predetermined threshold value, the comprehensive stability judgment unit 452 judges that the extracorporeal circulator 1 is not comprehensively stable. On the other hand, when the comprehensive stability determined by the comprehensive stability determination unit 451 is equal to or greater than the predetermined threshold value, the comprehensive stability judgment unit 452 judges that the extracorporeal circulator 1 is comprehensively stable.

The touch panel 52 is an example of the "display unit" of this disclosure, and is capable of displaying various kinds of information and detecting the contact of the finger of the operator or the like. The touch panel 52 transmits, to the control unit 40, information input by the operator or the like in response to an operation of the operator or the like.

The communication unit 53 communicates with the drive motor 4, the external monitor 16, the flow rate measurement unit 21, the arterial pressure measurement unit 37, and the pump rotation speed detection unit 36, and transmits and receives various kinds of information and various kinds of signals.

Here, in the auxiliary circulation operation using the extracorporeal circulator 1, one of necessary factors is to judge the timing suitable for the detachment of the extracorporeal circulator 1. That is, when the timing of the detachment of the extracorporeal circulator 1 is too early, the heart of the patient cannot withstand an operation, and the extracorporeal circulator 1 may be required to be reattached, or the patient may die. On the other hand, when the timing of the detachment of the extracorporeal circulator 1 is too late, complications such as bleeding may occur due to an anticoagulation therapy or consumption of blood plasma components. That is, there is an appropriate timing for the detachment of the extracorporeal circulator 1. Further, it is desirable that the timing suitable for the detachment of the extracorporeal circulator 1 can be determined early.

A criterion for judging the timing suitable for the detachment of the extracorporeal circulator 1 is, for example, the stability of the dynamic of the blood circulation in the circulation path 1R of the extracorporeal circulator 1, that is, the stability of the dynamic of the extracorporeal circulation. Another criterion for judging the timing suitable for the detachment of the extracorporeal circulator 1 is the stability of the heart function of the patient P, in other words, the degree of the recovery of the cardiac function of the patient P. The circulation stability indicating the stability of the dynamic of the blood circulation and the heart function stability indicating the stability of the heart function of the patient P will be further described with reference to the drawings.

First, the circulation stability will be described.

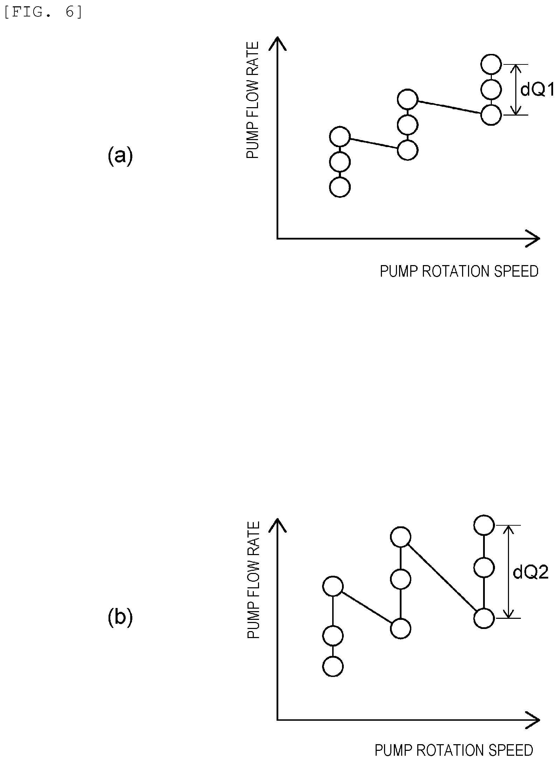

Fig. 6 shows graphs illustrating the circulation stability.
(a) of Fig. 6 is a graph illustrating a state in which the dynamic of the extracorporeal circulation is relatively stable. (b) of Fig. 6 is a graph illustrating a state in which the dynamic of the extracorporeal circulation is not relatively stable. Horizontal axes of the graphs shown in (a) of Fig. 6 and (b) of Fig. 6 represent the rotation speed of the centrifugal pump 3 detected by the pump rotation speed detection unit 36. Vertical axes of the graphs shown in (a) of Fig. 6 and (b) of Fig. 6 represent the measurement result of the flow rate measurement unit 21, that is, the flow rate of the blood sent out from the centrifugal pump 3 (pump blood transfer flow rate).

According to knowledge obtained by the present inventor, as shown in (a) of Fig. 6, when the dynamic of the extracorporeal circulation is relatively stable, in other words, when the circulation stability is relatively high, a variation width dQ1 of the pump blood transfer flow rate is relatively small at a constant rotation speed of the centrifugal pump 3. As shown in (a) of Fig. 6, a tendency that the variation width dQ1 of the pump blood transfer flow rate is relatively small is substantially the same regardless of the rotation speed of the centrifugal pump 3.

On the other hand, according to the knowledge obtained by the present inventor, as shown in (b) of Fig. 6, when the dynamic of the extracorporeal circulation is not relatively stable, in other words, when the circulation stability is relatively low, a variation width dQ2 of the pump blood transfer flow rate is relatively large at the constant rotation speed of the centrifugal pump 3. As shown in (b) of Fig. 6, a tendency that the variation width dQ2 of the pump blood transfer flow rate is relatively large is substantially the same regardless of the rotation speed of the centrifugal pump 3.

One of factors causing the circulation stability to be relatively low or factors causing the circulation stability to decrease is, for example, a blood removal failure caused by a distal portion of the blood removal side catheter 5 being suctioned against a blood vessel wall. When the blood removal failure occurs, a health care worker delivers, for example, a physiological salt solution to a vein or performs blood transfusion based on experience and knowledge. However, since supplement of a liquid such as a physiological salt solution, blood transfusion, or the like is not a method for eliminating a fundamental cause of the blood removal failure, the blood removal failure may occur again. Considering that the liquid such as a physiological salt solution may leak out of the blood vessel and cause edema, it is desirable to reduce an amount of the liquid such as a physiological salt solution to be supplemented or an amount of blood to be transfused. Thus, the stability of the dynamic of the extracorporeal circulation is often judged based on the experience and knowledge of the health care worker, and it may be difficult to judge the stability of the dynamic of the extracorporeal circulation with high accuracy.

Therefore, in the extracorporeal circulator 1 according to the present embodiment, the extracorporeal circulation management system 43 judges the stability of the dynamic of the extracorporeal circulation. That is, the control system 10 according to the present embodiment includes the extracorporeal circulation management system 43 that judges the stability of the dynamic of the extracorporeal circulation as one of criteria for judging the timing suitable for the detachment of the extracorporeal circulator 1. Therefore, the extracorporeal circulator 1 according to the present embodiment can judge the stability of the dynamic of the extracorporeal circulation with high accuracy, and can judge the timing suitable for the detachment of the extracorporeal circulator 1 with high accuracy.

Next, the heart function stability will be described.

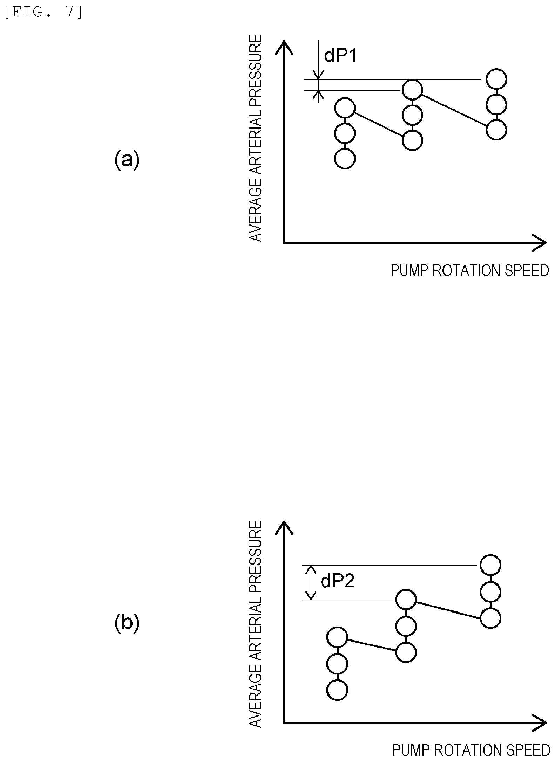

Fig. 7 shows graphs illustrating the heart function stability.
(a) of Fig. 7 is a graph illustrating a state in which the heart function of the patient P is relatively stable. (b) of Fig. 7 is a graph illustrating a state in which the heart function of the patient P is not relatively stable. Horizontal axes of the graphs shown in (a) of Fig. 7 and (b) of Fig. 7 represent the rotation speed of the centrifugal pump 3 detected by the pump rotation speed detection unit 36. Vertical axes of the graphs shown in (a) of Fig. 7 and (b) of Fig. 7 represent the measurement result of the arterial pressure measurement unit 37, that is, the pressure of the blood flowing through the artery of the patient P (average arterial pressure in (a) of Fig. 7 and (b) of Fig. 7).

According to the knowledge obtained by the inventor, as shown in (a) of Fig. 7, when the heart function of the patient P is relatively stable, in other words, when the heart function stability is relatively high, a change width dP1 between the average arterial pressures of the patient P is relatively small at different rotation speeds of the centrifugal pump 3. That is, when the degree of the recovery of the cardiac function of the patient P is relatively high and a degree of dependence of the patient P on the extracorporeal circulator 1 is relatively low, the change width dP1 between the average arterial pressures of the patient P is relatively small even when the rotation speed of the centrifugal pump 3 is lowered.

On the other hand, according to the knowledge obtained by the inventor, as shown in (b) of Fig. 7, when the heart function of the patient P is not relatively stable, in other words, when the heart function stability is relatively low, a change width dP2 between the average arterial pressures of the patient P is relatively large at different rotation speeds of the centrifugal pump 3. That is, in a case where the degree of the recovery of the cardiac function of the patient P is relatively low and the degree of the dependence of the patient P on the extracorporeal circulator 1 is relatively high, when the rotation speed of the centrifugal pump 3 is lowered, the change width dP2 between the average arterial pressures of the patient P is relatively large.

Therefore, in the extracorporeal circulator 1 according to the present embodiment, the cardiac function measurement system 44 judges the stability of the heart function of the patient P. That is, the control system 10 according to the present embodiment includes the cardiac function measurement system 44 that judges the stability of the heart function of the patient P as one of the criteria for judging the timing suitable for the detachment of the extracorporeal circulator 1. Therefore, the extracorporeal circulator 1 according to the present embodiment can judge the stability of the heart function of the patient P with high accuracy, and can judge the timing suitable for the detachment of the extracorporeal circulator 1 with high accuracy.

Next, a specific example of operation of the extracorporeal circulator 1 according to the present embodiment will be described with reference to the drawings.

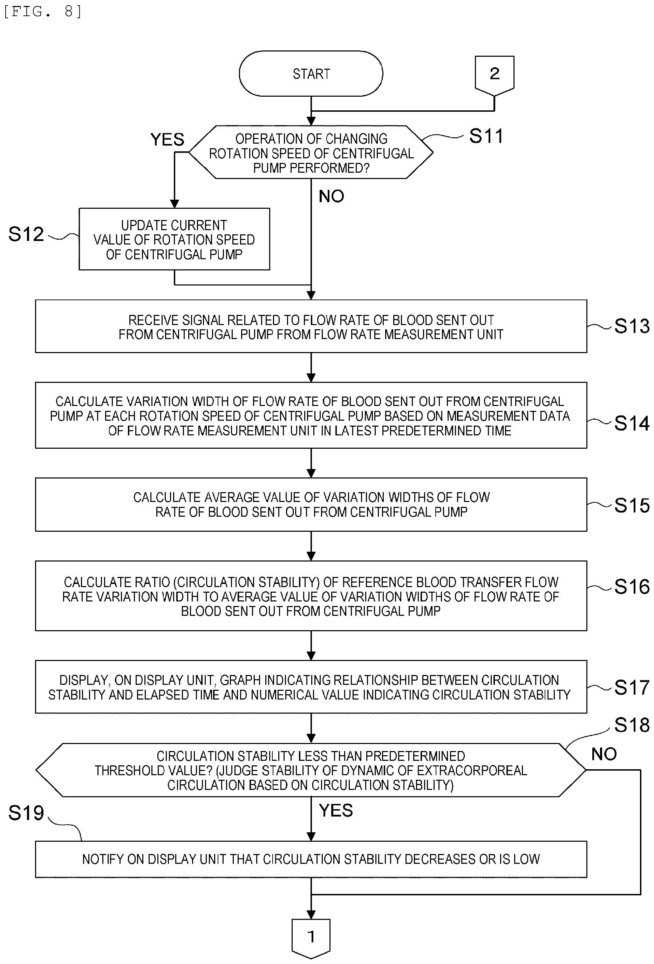

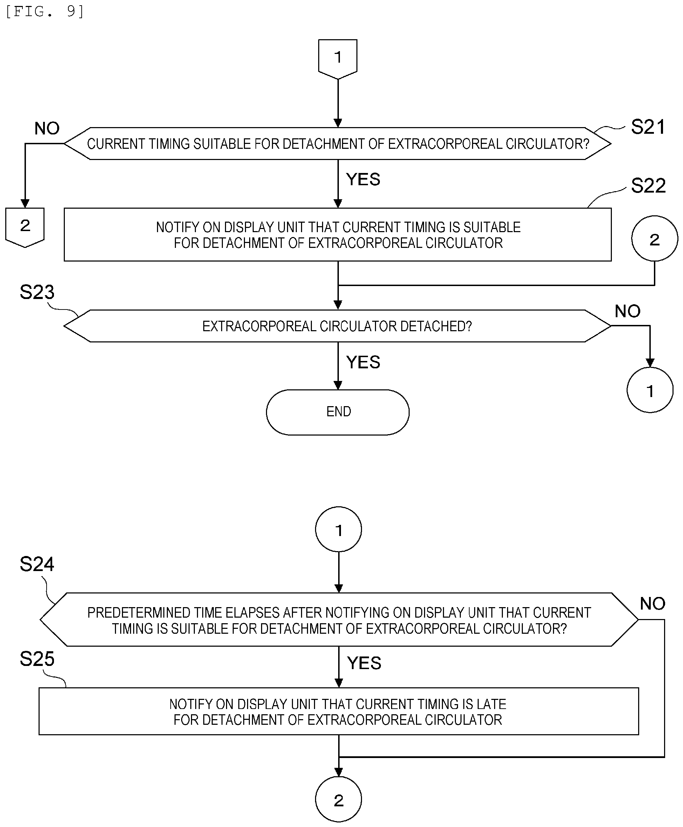

Figs. 8 and 9 are flowcharts illustrating a first specific example of the operation of the extracorporeal circulator according to the present embodiment.

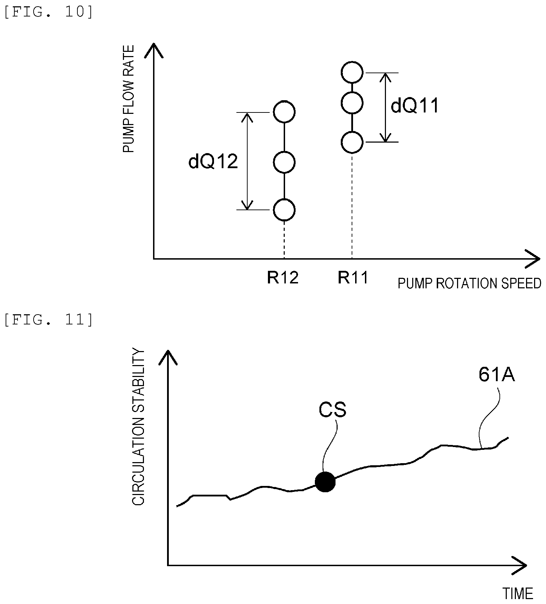

Fig. 10 is a graph illustrating an example of a relationship between a rotation speed of a centrifugal pump and a blood transfer flow rate of the centrifugal pump.

Fig. 11 is a graph illustrating an example of the relationship between the circulation stability and the elapsed time.

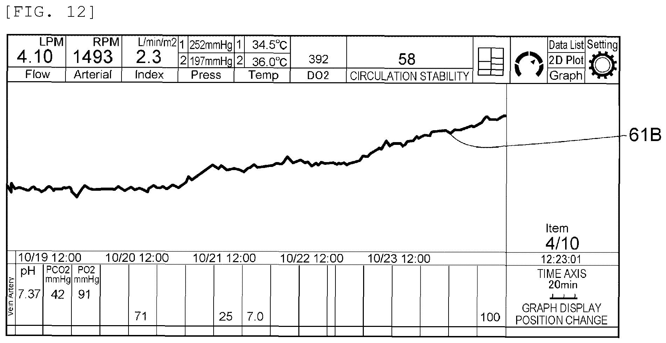

Fig. 12 is a schematic diagram illustrating an example of a screen on which both a graph indicating the relationship between the circulation stability and the elapsed time and the numerical value indicating the circulation stability are displayed on the display unit.

First, in step S11 shown in Fig. 8, the control unit 40 of the control system 10 judges whether an operation of changing the rotation speed of the centrifugal pump 3 is performed. That is, the control unit 40 judges whether the rotation speed of the centrifugal pump 3 is changed based on the signal G related to the rotation speed of the centrifugal pump 3 detected by the pump rotation speed detection unit 36. When the operation of changing the rotation speed of the centrifugal pump 3 is performed (step S11: YES), the control unit 40 updates a current value of the rotation speed of the centrifugal pump 3 in step S12. When the operation of changing the rotation speed of the centrifugal pump 3 is not performed (step S11: NO), in step S13, the control unit 40 receives the signal S4 related to the flow rate (pump blood transfer flow rate) of the blood sent out from the centrifugal pump 3 from the flow rate measurement unit 21.

Subsequently, in step S14, the pump blood transfer flow rate variation width determination unit 431 of the extracorporeal circulation management system 43 calculates the variation width (pump blood transfer flow rate variation width) of the flow rate of the blood sent out from the centrifugal pump 3 at each rotation speed of the centrifugal pump 3 based on the measurement data of the flow rate measurement unit 21 in the latest predetermined time. For example, in the example shown in Fig. 10, the pump blood transfer flow rate variation width determination unit 431 calculates a pump blood transfer flow rate variation width dQ11 at a rotation speed R11 of the centrifugal pump 3 based on the measurement data of the flow rate measurement unit 21 in the latest predetermined time. The pump blood transfer flow rate variation width determination unit 431 calculates a pump blood transfer flow rate variation width dQ12 at a rotation speed R12 of the centrifugal pump 3 based on the measurement data of the flow rate measurement unit 21 in the latest predetermined time.

Subsequently, in step S15, the pump blood transfer flow rate variation width determination unit 431 calculates the average value of the variation widths of the flow rate of the blood sent out from the centrifugal pump 3. For example, in the example shown in Fig. 10, the pump blood transfer flow rate variation width determination unit 431 calculates (dQ11 + dQ12)/2 as the average value of the pump blood transfer flow rate variation widths.

Subsequently, in step S16, the circulation stability determination unit 432 calculates a ratio (circulation stability) CS (see Fig. 11) of the reference blood transfer flow rate variation width to the average value of the pump blood transfer flow rate variation widths calculated in step S14. The reference blood transfer flow rate variation width is as described above with reference to Fig. 5, and is, for example, 0.7 L/min in the specific example. Thus, for example, in the example shown in Fig. 10, the circulation stability determination unit 432 calculates 0.7 × 100/((dQ11 + dQ12)/2) as the ratio (circulation stability) CS of the reference blood transfer flow rate variation width to the average value of the pump blood transfer flow rate variation widths.

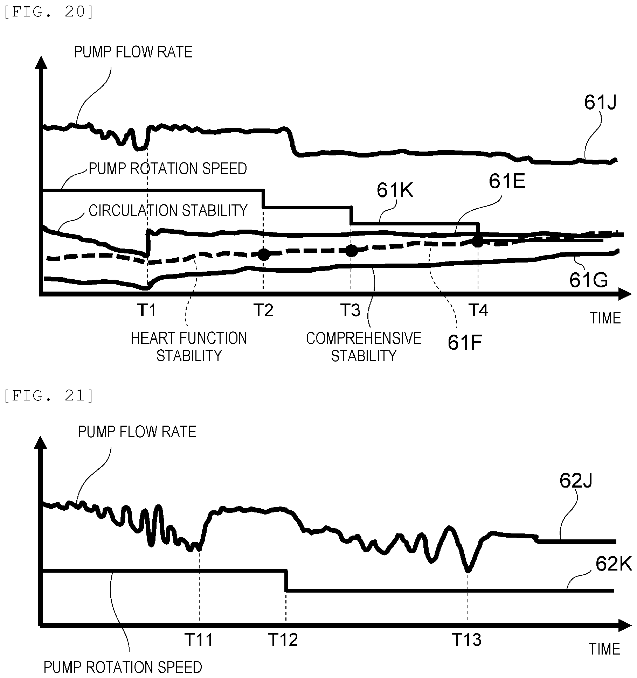

Subsequently, in step S17, the display processing unit 41 displays, on the external monitor 16, a graph indicating a relationship between the circulation stability CS and the elapsed time and a numerical value indicating the circulation stability CS. Examples of the graph showing the relationship between the circulation stability CS and the elapsed time include a graph 61A shown in Fig. 11 and a graph 61B shown in Fig. 12. The graph showing the relationship between the circulation stability CS and the elapsed time is not limited to the graph 61A shown in Fig. 11 and the graph 61B shown in Fig. 12. In the example of the screen display shown in Fig. 12, the graph 61B showing the relationship between the circulation stability CS and the elapsed time, a graph (see Fig. 20) showing a relationship between a blood transfer flow rate of the centrifugal pump 3 and the elapsed time, and a graph (Fig. 20) showing a relationship between the rotation speed of the centrifugal pump 3 and the elapsed time may be displayed. This will be described later in detail with reference to Fig. 20.

An example of the numerical value indicating the circulation stability CS is displayed as "58" on a display screen shown in Fig. 12. That is, for example, it is assumed that the pump blood transfer flow rate variation width dQ11 at the rotation speed R11 of the centrifugal pump 3 is 1.1 L/min. Further, it is assumed that the pump blood transfer flow rate variation width dQ12 at the rotation speed R12 of the centrifugal pump 3 is 1.3 L/min. In this case, the average value of the pump blood transfer flow rate variation widths is 1.2 L/min (= (1.1 + 1.3)/2). Thus, in the specific example, since the reference blood transfer flow rate variation width is 0.7 L/min, the numerical value indicating the circulation stability CS is "58 (approximately equal to 0.7 × 100/1.2)". The numerical value indicating the circulation stability CS is not limited to "58".

Subsequently, in step S18, the circulation stability judgment unit 433 judges the stability of the dynamic of the extracorporeal circulation based on the circulation stability CS determined by the circulation stability determination unit 432. That is, the circulation stability judgment unit 433 judges whether the circulation stability CS determined by the circulation stability determination unit 432 is less than the predetermined threshold value. When the circulation stability CS determined by the circulation stability determination unit 432 is less than the predetermined threshold value (step S18: YES), the circulation stability judgment unit 433 judges that the dynamic of the extracorporeal circulation is not stable. Further, in step S19, the notification processing unit 42 performs notification on the external monitor 16 that the circulation stability CS decreases or is low.

On the other hand, when the circulation stability CS determined by the circulation stability determination unit 432 is equal to or greater than the predetermined threshold value (step S18: NO), the circulation stability judgment unit 433 judges that the dynamic of the extracorporeal circulation is stable. Further, in step S21 shown in Fig. 9, the control unit 40 judges the timing suitable for the detachment of the extracorporeal circulator 1 using a judgment result of the circulation stability judgment unit 433 as one of the criteria. That is, the control unit 40 judges whether the current timing is suitable for the detachment of the extracorporeal circulator 1.

When the current timing is suitable for the detachment of the extracorporeal circulator 1 (step S21: YES), in step S22, the notification processing unit 42 performs notification on the external monitor 16 that the current timing is suitable for the detachment of the extracorporeal circulator 1. On the other hand, when the current timing is not suitable for the detachment of the extracorporeal circulator 1 (step S21: NO), the process described above with respect to step S11 is executed. In step S23 following step S22, the control unit 40 judges whether the extracorporeal circulator 1 is detached from the patient P. When the extracorporeal circulator 1 is detached from the patient P (step S23: YES), the control unit 40 ends the operation of the extracorporeal circulator 1.

On the other hand, when the extracorporeal circulator 1 is not detached from the patient P (step S23: NO), in step S24, the control unit 40 judges whether predetermined time elapses after the notification processing unit 42 performs notification on the external monitor 16 that the current timing is suitable for the detachment of the extracorporeal circulator 1. For example, the control unit 40 measures, by using a timer function of the control unit 40, time elapsed after the notification processing unit 42 performs notification on the external monitor 16 that the current timing is suitable for the detachment of the extracorporeal circulator 1.

When the predetermined time elapses after the notification processing unit 42 performs notification on the external monitor 16 that the current timing is suitable for the detachment of the extracorporeal circulator 1 (step S24: YES), the notification processing unit 42 performs notification on the external monitor 16 that the current timing is late for the detachment of the extracorporeal circulator 1 in step S25. For example, the notification processing unit 42 pops up and displays on the external monitor 16 using characters, figures, symbols, and the like that the current timing is late for the detachment of the extracorporeal circulator 1. Alternatively, the notification processing unit 42 may performs notification that the current timing is late for the detachment of the extracorporeal circulator 1 by voice.

On the other hand, when the predetermined time does not elapse after the notification processing unit 42 performs notification on the external monitor 16 that the current timing is suitable for the detachment of the extracorporeal circulator 1 (step S24: NO), the process described above with respect to step S23 is executed.

According to the extracorporeal circulator 1 of the specific example, the extracorporeal circulation management system 43 determines the circulation stability CS based on the reference blood transfer flow rate variation width and the pump blood transfer flow rate variation width, and judges the stability of the dynamic of the extracorporeal circulation based on the circulation stability CS. Accordingly, the control system 10 of the extracorporeal circulator 1 according to the specific example can judge the stability of the dynamic of the extracorporeal circulation with high accuracy, and can judge the timing suitable for the detachment of the extracorporeal circulator 1 with high accuracy.

In addition, the control system 10 of the extracorporeal circulator 1 according to the specific example can judge the stability of the dynamic of the extracorporeal circulation with high accuracy, and thus can capture a sign of a decrease in the stability of the dynamic of the extracorporeal circulation. Therefore, for example, the health care worker can adjust a position of the distal portion of the blood removal side catheter 5, supplement the liquid such as a physiological salt solution, transfuse blood, or the like at a more appropriate timing, and can prevent the decrease in the stability of the dynamic of the extracorporeal circulation. Accordingly, the amount of the liquid such as a physiological salt solution to be supplemented and the amount of the blood to be transfused can be reduced.

By checking the external monitor 16, the health care worker can easily check the circulation stability CS indicating the stability of the dynamic of the extracorporeal circulation, and can visually grasp the timing suitable for the detachment of the extracorporeal circulator 1. By checking the external monitor 16, for example, the health care worker can look back on interventions or procedures such as adjustment of the position of the distal portion of the blood removal side catheter 5, supplementation of the liquid such as a physiological salt solution, and blood transfusion.

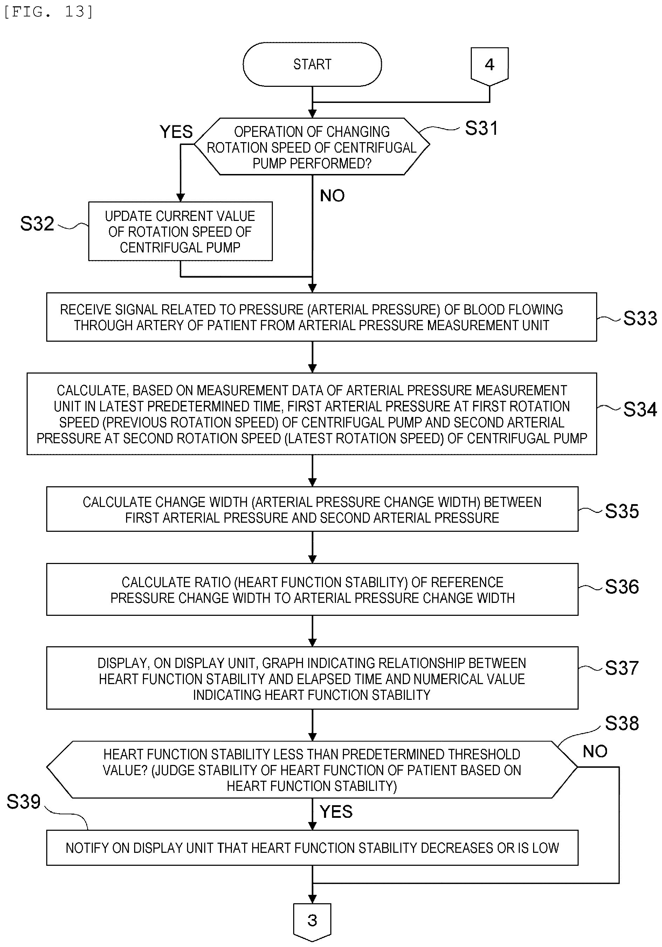

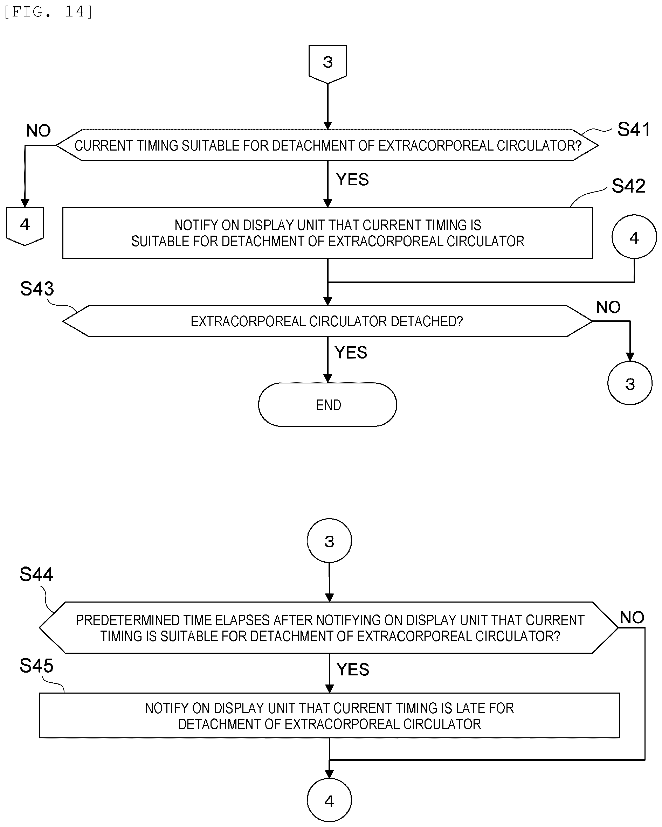

Figs. 13 and 14 are flowcharts illustrating a second specific example of the operation of the extracorporeal circulator according to the present embodiment.

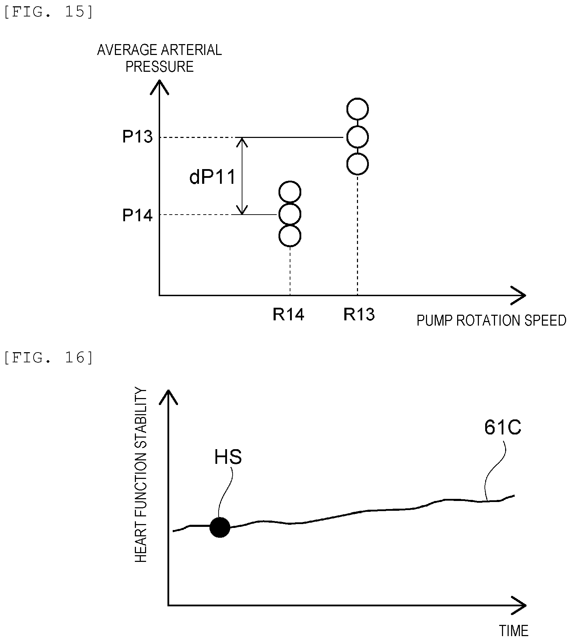

Fig. 15 is a graph illustrating an example of a relationship between the rotation speed of the centrifugal pump and the average arterial pressure.

Fig. 16 is a graph illustrating an example of the relationship between the heart function stability and the elapsed time.

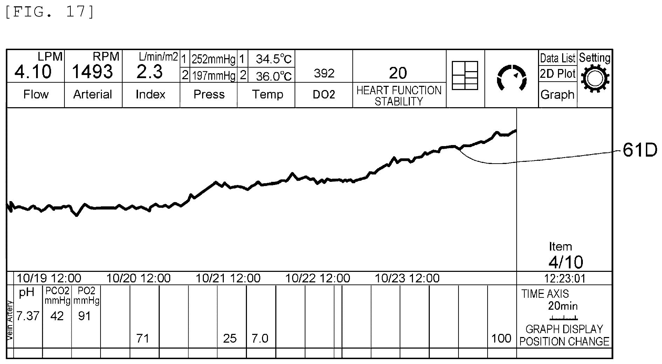

Fig. 17 is a schematic diagram illustrating an example of a screen on which both a graph showing the relationship between the heart function stability and the elapsed time and a numerical value showing the heart function stability are displayed on the display unit.

First, processes of step S31 and step S32 shown in Fig. 13 are the same as the processes of step S11 and step S12 described above with reference to Fig. 8. In step S33 following step S31 and step S32, the control unit 40 receives the signal S7 related to the pressure (arterial pressure) of the blood flowing through the artery of the patient P from the arterial pressure measurement unit 37.

Subsequently, in step S34, the arterial pressure change width determination unit 441 of the cardiac function measurement system 44 calculates, based on the measurement data of the arterial pressure measurement unit 37 in the latest predetermined time, the first arterial pressure of the blood flowing through the artery of the patient P at the first rotation speed (previous rotation speed) of the centrifugal pump 3 detected by the pump rotation speed detection unit 36 and the second arterial pressure of the blood flowing through the artery of the patient P at the second rotation speed (latest rotation speed) of the centrifugal pump 3 detected by the pump rotation speed detection unit 36. For example, in the example shown in Fig. 15, the arterial pressure change width determination unit 441 calculates a first average arterial pressure P13 as the first arterial pressure at a first rotation speed R13 based on the measurement data of the arterial pressure measurement unit 37 in the latest predetermined time. The arterial pressure change width determination unit 441 calculates a second average arterial pressure P14 as the second arterial pressure at a second rotation speed R14 based on the measurement data of the arterial pressure measurement unit 37 in the latest predetermined time.

Subsequently, in step S35, the arterial pressure change width determination unit 441 calculates a change width (arterial pressure change width) between the first arterial pressure and the second arterial pressure. For example, in the example shown in Fig. 15, the arterial pressure change width determination unit 441 calculates a change width (arterial pressure change width) dP11 (= P13 - P14) between the first average arterial pressure P13 and the second average arterial pressure P14.

Subsequently, in step S36, the heart function stability determination unit 442 calculates a ratio (heart function stability) HS (see Fig. 16) of the reference pressure change width to the arterial pressure change width dP11 calculated in step S35. The reference pressure change width is as described above with reference to Fig. 5, and is, for example, 10 mmHg in the specific example. Thus, for example, in the example shown in Fig. 15, the heart function stability determination unit 442 calculates 10 × 100/dP11 as the ratio (heart function stability) HS of the reference pressure change width to the arterial pressure change width dP11.

Subsequently, in step S37, the display processing unit 41 displays, on the external monitor 16, a graph indicating a relationship between the heart function stability HS and the elapsed time and a numerical value indicating the heart function stability HS. Examples of the graph showing the relationship between the heart function stability HS and the elapsed time are, for example, a graph 61C shown in Fig. 16 and a graph 61D shown in Fig. 17. The graph showing the relationship between the heart function stability HS and the elapsed time is not limited to the graph 61C shown in Fig. 16 and the graph 61D shown in Fig. 17. In the example of the screen display shown in Fig. 17, the graph 61D showing the relationship between the heart function stability HS and the elapsed time, the graph (see Fig. 20) showing the relationship between the blood transfer flow rate of the centrifugal pump 3 and the elapsed time, and the graph (Fig. 20) showing the relationship between the rotation speed of the centrifugal pump 3 and the elapsed time may be displayed. This will be described later in detail with reference to Fig. 20.

An example of the numerical value indicating the heart function stability HS is displayed as "20" on a display screen shown in Fig. 17. That is, for example, it is assumed that the first average arterial pressure P13 at the first rotation speed of the centrifugal pump 3 is 100 mmHg. It is assumed that the second average arterial pressure P14 at the second rotation speed of the centrifugal pump 3 is 50 mmHg. In this case, the arterial pressure change width dP11 is 50 mmHg (= 100 - 50). Thus, in the specific example, since the reference pressure change width is 10 mmHg, the numerical value indicating the heart function stability HS is "20 (= 10 × 100/50)". The numerical value indicating the heart function stability HS is not limited to "20".

Subsequently, in step S38, the heart function stability judgment unit 443 judges the stability of the heart function of the patient P based on the heart function stability HS determined by the heart function stability determination unit 442. That is, the heart function stability judgment unit 443 judges whether the heart function stability HS determined by the heart function stability determination unit 442 is less than the predetermined threshold value. When the heart function stability HS determined by the heart function stability determination unit 442 is less than the predetermined threshold value (step S38: YES), the heart function stability judgment unit 443 judges that the heart function of the patient P is not stable, that is, the degree of the recovery of the cardiac function of the patient P is low. Further, in step S39, the notification processing unit 42 performs notification on the external monitor 16 that the heart function stability HS decreases or is low.

On the other hand, when the heart function stability HS determined by the heart function stability determination unit 442 is equal to or greater than the predetermined threshold value (step S38: NO), the heart function stability judgment unit 443 judges that the heart function of the patient P is stable, that is, the degree of the recovery of the cardiac function of the patient P is high. Further, in step S41 shown in Fig. 14, the control unit 40 judges the timing suitable for the detachment of the extracorporeal circulator 1 using a judgment result of the heart function stability judgment unit 443 as one of the criteria. That is, the control unit 40 judges whether the current timing is suitable for the detachment of the extracorporeal circulator 1. Subsequently, processes of steps S41 to S45 are the same as the processes of steps S21 to S25 described above with reference to Fig. 9.

According to the extracorporeal circulator 1 of the specific example, the cardiac function measurement system 44 determines the heart function stability HS based on the reference pressure change width and the arterial pressure change width dP11, and judges the stability of the heart function of the patient P based on the heart function stability HS. Accordingly, the control system 10 of the extracorporeal circulator 1 according to the specific example can judge the stability of the heart function of the patient P with high accuracy, and can judge the timing suitable for the detachment of the extracorporeal circulator 1 with high accuracy.

Since the control system 10 of the extracorporeal circulator 1 according to the specific example can judge the stability of the heart function of the patient P with high accuracy, it is possible to prevent the timing of the detachment of the extracorporeal circulator 1 from being too early or too late, to optimize attachment time of the extracorporeal circulator 1, and to reduce the occurrence of the complications.

By checking the external monitor 16, the health care worker can easily check the heart function stability HS indicating the stability of the heart function of the patient P and visually grasp the timing suitable for the detachment of the extracorporeal circulator 1.

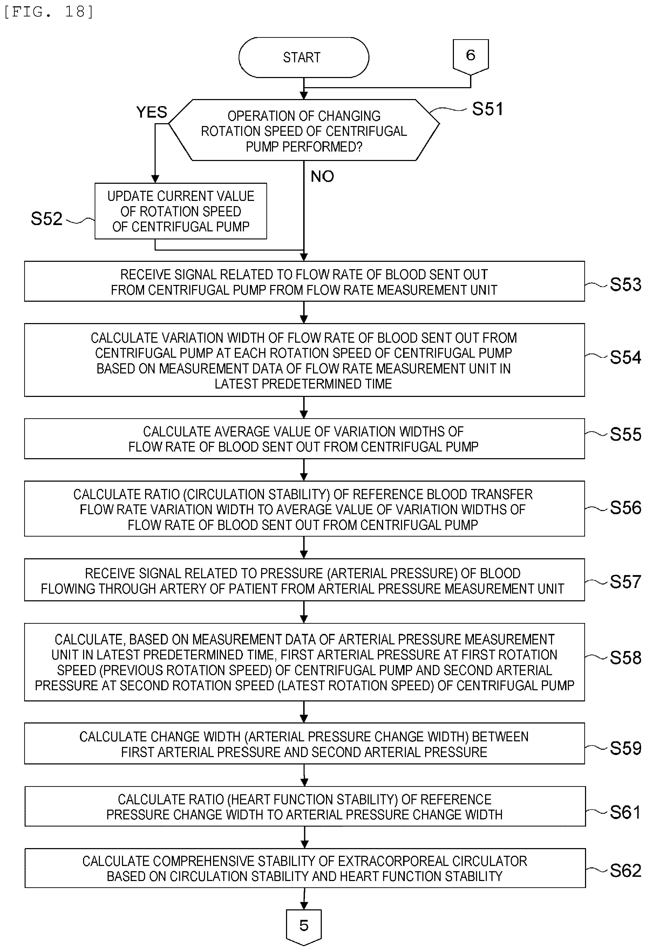

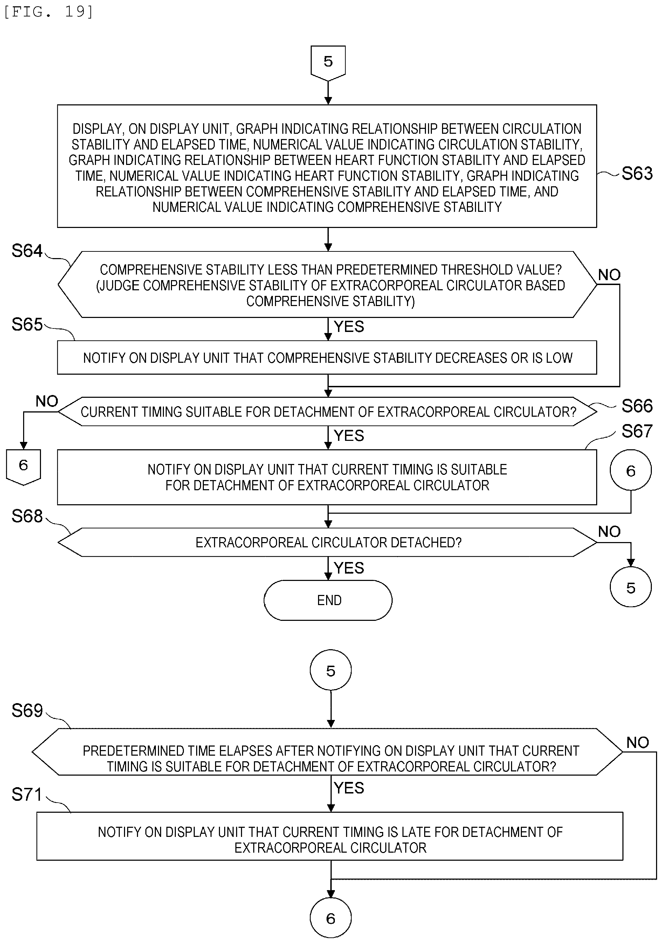

Figs. 18 and 19 are flowcharts illustrating a third specific example of the operation of the extracorporeal circulator according to the present embodiment.

Fig. 20 is a timing chart illustrating operation of the specific example.

Fig. 21 is a timing chart illustrating operation of a comparative example.

First, processes of steps S51 to S56 shown in Fig. 18 are the same as the processes of steps S11 to S16 described above with reference to Fig. 8. Processes of steps S57 to S61 shown in Fig. 18 are the same as the processes of steps S33 to S36 described above with reference to Fig. 13.

In step S62 following step S61, the comprehensive stability determination unit 451 of the comprehensive judgment system 45 calculates comprehensive stability TS of the extracorporeal circulator 1 based on the circulation stability CS determined by the circulation stability determination unit 432 and the heart function stability HS determined by the heart function stability determination unit 442. For example, the comprehensive stability determination unit 451 calculates (circulation stability CS × m + heart function stability HS × n)/(m + n) as the comprehensive stability TS.

Subsequently, in step S63, the display processing unit 41 displays, on the external monitor 16, the graph indicating the relationship between the circulation stability CS and the elapsed time, the numerical value indicating the circulation stability CS, the graph indicating the relationship between the heart function stability HS and the elapsed time, the numerical value indicating the heart function stability HS, a graph indicating a relationship between the comprehensive stability TS and the elapsed time, and a numerical value indicating the comprehensive stability TS. An example of the graph showing the relationship between the circulation stability CS and the elapsed time is, for example, a graph 61E shown in Fig. 20. An example of the graph showing the relationship between the heart function stability HS and the elapsed time is, for example, a graph 61F shown in Fig. 20. An example of the graph showing the relationship between the comprehensive stability TS and the elapsed time is, for example, a graph 61G shown in Fig. 20. For example, in the example shown in Fig. 20, each of the coefficient m and the coefficient n described above is "1". Then, the comprehensive stability determination unit 451 calculates (circulation stability CS + heart function stability HS)/2 as the comprehensive stability TS.

Subsequently, in step S64, the comprehensive stability judgment unit 452 judges the comprehensive stability of the extracorporeal circulator 1 based on the comprehensive stability TS determined by the comprehensive stability determination unit 451. That is, the comprehensive stability judgment unit 452 judges whether the comprehensive stability TS determined by the comprehensive stability determination unit 451 is less than the predetermined threshold value. When the comprehensive stability TS determined by the comprehensive stability determination unit 451 is less than the predetermined threshold value (step S64: YES), the comprehensive stability judgment unit 452 judges that the extracorporeal circulator 1 is not comprehensively stable. Further, in step S65, the notification processing unit 42 performs notification on the external monitor 16 that the comprehensive stability TS decreases or is low.

On the other hand, when the comprehensive stability TS determined by the comprehensive stability determination unit 451 is equal to or greater than the predetermined threshold value (step S64: NO), the comprehensive stability judgment unit 452 judges that the extracorporeal circulator 1 is comprehensively stable. Further, in step S66, the control unit 40 judges the timing suitable for the detachment of the extracorporeal circulator 1 using a judgment result of the comprehensive stability judgment unit 452 as one of the criteria. Subsequently, processes of steps S66 to S71 are the same as the processes of steps S21 to S25 described above with reference to Fig. 9.

In the comparative example shown in Fig. 21, for example, a graph 62J showing the relationship between the blood transfer flow rate of the centrifugal pump 3 and the elapsed time and a graph 62K showing the relationship between the rotation speed of the centrifugal pump 3 and the elapsed time are displayed on the external monitor 16, while the graph showing the relationship between the circulation stability CS and the elapsed time, the graph showing the relationship between the heart function stability HS and the elapsed time, and the graph showing the relationship between the comprehensive stability TS and the elapsed time are not displayed on the external monitor 16. Thus, even when the health care worker notices that the blood transfer flow rate of the centrifugal pump 3 decreases and becomes unstable at a timing T11 shown in Fig. 21 and supplements the liquid such as a physiological salt solution or transfuses blood based on experience and knowledge, the blood transfer flow rate of the centrifugal pump 3 decreases again and becomes unstable when the rotation speed of the centrifugal pump 3 is decreased at a timing T12 shown in Fig. 21, and it is necessary to supplement the liquid such as a physiological salt solution or transfuse blood at a timing T13 shown in Fig. 21.

In contrast, according to the extracorporeal circulator 1 of the specific example, as shown in Fig. 20, the display processing unit 41 displays, on the external monitor 16, a graph 61J indicating the relationship between the blood transfer flow rate of the centrifugal pump 3 and the elapsed time, a graph 61K indicating the relationship between the rotation speed of the centrifugal pump 3 and the elapsed time, the graph 61E indicating the relationship between the circulation stability CS and the elapsed time, the graph 61F indicating the relationship between the heart function stability HS and the elapsed time, and the graph 61G indicating the relationship between the comprehensive stability TS and the elapsed time.

Therefore, at a timing T1 shown in Fig. 20, the health care worker can notice that the blood transfer flow rate of the centrifugal pump 3 decreases and becomes unstable and the circulation stability CS decreases. Therefore, at the timing T1 shown in Fig. 20, the health care worker can not only supplement the solution such as a physiological salt solution or transfuse blood, but also adjust the position of the distal portion of the blood removal side catheter 5. In this way, the health care worker can solve the fundamental cause of the decrease in the circulation stability CS due to, for example, the blood removal failure. Therefore, at timings T2, T3, and T4 shown in Fig. 20, even when the health care worker decreases the rotation speed of the centrifugal pump 3 in a stepwise manner, the blood transfer flow rate of the centrifugal pump 3 can be prevented from decreasing and becoming unstable again. Further, as in the graph 61F showing the relationship between the heart function stability HS and the elapsed time and the graph 61G showing the relationship between the comprehensive stability TS and the elapsed time shown in Fig. 20, even when the health care worker decreases the rotation speed of the centrifugal pump 3 in the stepwise manner, the decrease in the heart function stability HS and the comprehensive stability TS can be prevented. Accordingly, the extracorporeal circulator 1 according to the specific example can judge the timing suitable for the detachment of the extracorporeal circulator 1 with high accuracy.

According to the extracorporeal circulator 1 of the specific example, the control system 10 can judge both the stability of the dynamic of the extracorporeal circulation and the stability of the heart function of the patient P with high accuracy, and can judge the timing suitable for the detachment of the extracorporeal circulator 1 with higher accuracy.

By checking the external monitor 16, the health care worker can easily check the circulation stability CS indicating the stability of the dynamic of the extracorporeal circulation, the heart function stability HS indicating the stability of the heart function of the patient P, and the comprehensive stability TS indicating the comprehensive stability of the extracorporeal circulator 1, and can more reliably and visually grasp the timing suitable for the detachment of the extracorporeal circulator 1.

The embodiment according to this disclosure has been described above. However, this disclosure is not limited to the above-described embodiment, and various modifications can be made without departing from the scope of the claims. A part of configurations of the above-described embodiment can be omitted, or the configurations of the above-described embodiment can be freely combined to be different from those described above.

### Reference Signs List

1: extracorporeal circulator
1R: circulation path
2: artificial lung
3: centrifugal pump
4: drive motor
5: blood removal side catheter
6: blood transfer side catheter
8a, 8b, 9a, 9b: connector
10: control system
11: blood removal tube
12: blood transfer tube
14: oxygen supply tube
16: external monitor
21: flow rate measurement unit
30: storage unit
31: program
32: pump characteristic storage unit
33: reference blood transfer flow rate variation width storage unit
34: reference pressure change width storage unit
36: pump rotation speed detection unit
37: arterial pressure measurement unit
40: control unit
41: display processing unit
42: notification processing unit
43: extracorporeal circulation management system
44: cardiac function measurement system
45: comprehensive judgment system
48: CPU
49: FPGA
51: computer
52: touch panel
53: communication unit
61A, 61B, 61C, 61D, 61E, 61F, 61G, 61J, 61K, 62J, 62K: graph
431: pump blood transfer flow rate variation width determination unit
432: circulation stability determination unit
433: circulation stability judgment unit
441: arterial pressure change width determination unit
442: heart function stability determination unit
443: heart function stability judgment unit
451: comprehensive stability determination unit
452: comprehensive stability judgment unit
CS: circulation stability
G: signal
HS: heart function stability
P: patient
P1: heart
P13: first average arterial pressure
P14: second average arterial pressure
R11, R12: rotation speed
R13: first rotation speed
R14: second rotation speed
S1, S2, S3, S4, S5, S7: signal
SG: command
TS: comprehensive stability
dP1: change width
dP11: arterial pressure change width
dP2: change width
dQ1: variation width
dQ11, dQ12: pump blood transfer flow rate variation width
dQ2: variation width

## Claims

1. An extracorporeal circulator for extracorporeal circulation of blood using a circulation path, the extracorporeal circulator comprising:
a blood removal side catheter configured to have a part inserted into a patient and guide the blood taken from the patient;
a pump configured to take the blood from the patient and return the blood to the patient;
a blood transfer side catheter provided downstream of the pump, and configured to have a part inserted into the patient and guide the blood sent out from the pump to the patient;
a pump rotation speed detection unit configured to detect a rotation speed of the pump; and
a control system including at least one of an extracorporeal circulation management system configured to judge stability of a dynamic of the extracorporeal circulation and a cardiac function measurement system configured to judge stability of a heart function of the patient.

2. The extracorporeal circulator according to claim 1, wherein
the extracorporeal circulation management system includes
a pump blood transfer flow rate variation width determination unit configured to determine, based on a measurement result of a flow rate measurement unit provided in the circulation path, a pump blood transfer flow rate variation width indicating a variation width of a flow rate of the blood sent out from the pump at the rotation speed detected by the pump rotation speed detection unit,
a circulation stability determination unit configured to determine circulation stability indicating the stability of the dynamic of the extracorporeal circulation based on a reference blood transfer flow rate variation width indicating a standard variation width of the flow rate of the blood sent out from the pump and the pump blood transfer flow rate variation width determined by the pump blood transfer flow rate variation width determination unit, and
a circulation stability judgment unit configured to judge the stability of the dynamic of the extracorporeal circulation based on the circulation stability determined by the circulation stability determination unit.

3. The extracorporeal circulator according to claim 2, wherein
the control system further includes a display processing unit configured to display, on a display unit, at least one of a graph indicating a relationship between the circulation stability and elapsed time and a numerical value indicating the circulation stability.

4. The extracorporeal circulator according to claim 1, wherein
the cardiac function measurement system includes
an arterial pressure change width determination unit configured to determine, based on a measurement result of an arterial pressure measurement unit provided in the patient, an arterial pressure change width indicating a change width between a first arterial pressure of the blood flowing through an artery of the patient at a first rotation speed of the pump detected by the pump rotation speed detection unit and a second arterial pressure of the blood flowing through the artery of the patient at a second rotation speed of the pump detected by the pump rotation speed detection unit,
a heart function stability determination unit configured to determine heart function stability indicating the stability of the heart function of the patient based on a reference pressure change width indicating a standard change width between the first arterial pressure and the second arterial pressure and the arterial pressure change width determined by the arterial pressure change width determination unit, and
a heart function stability judgment unit configured to judge the stability of the heart function of the patient based on the heart function stability determined by the heart function stability determination unit.

5. The extracorporeal circulator according to claim 4, wherein
the control system further includes a display processing unit configured to display, on a display unit, at least one of a graph indicating a relationship between the heart function stability and elapsed time and a numerical value indicating the heart function stability.

6. The extracorporeal circulator according to claim 1, wherein
the control system includes both the extracorporeal circulation management system and the cardiac function measurement system, and includes a comprehensive judgment system configured to judge stability of the entire extracorporeal circulator,
the extracorporeal circulation management system includes
a pump blood transfer flow rate variation width determination unit configured to determine, based on a measurement result of a flow rate measurement unit provided in the circulation path, a pump blood transfer flow rate variation width indicating a variation width of a flow rate of the blood sent out from the pump at the rotation speed detected by the pump rotation speed detection unit, and
a circulation stability determination unit configured to determine circulation stability indicating the stability of the dynamic of the extracorporeal circulation based on a reference blood transfer flow rate variation width indicating a standard variation width of the flow rate of the blood sent out from the pump and the pump blood transfer flow rate variation width determined by the pump blood transfer flow rate variation width determination unit,
the cardiac function measurement system includes
an arterial pressure change width determination unit configured to determine, based on a measurement result of an arterial pressure measurement unit provided in the patient, an arterial pressure change width indicating a change width between a first arterial pressure of the blood flowing through an artery of the patient at a first rotation speed of the pump detected by the pump rotation speed detection unit and a second arterial pressure of the blood flowing through the artery of the patient at a second rotation speed of the pump detected by the pump rotation speed detection unit, and
a heart function stability determination unit configured to determine heart function stability indicating the stability of the heart function of the patient based on a reference pressure change width indicating a standard change width between the first arterial pressure and the second arterial pressure and the arterial pressure change width determined by the arterial pressure change width determination unit, and
the comprehensive judgment system includes
a comprehensive stability determination unit configured to determine comprehensive stability indicating comprehensive stability of the extracorporeal circulator based on the circulation stability determined by the circulation stability determination unit and the heart function stability determined by the heart function stability determination unit, and
a comprehensive stability judgment unit configured to judge the comprehensive stability of the extracorporeal circulator based on the comprehensive stability determined by the comprehensive stability determination unit.

7. The extracorporeal circulator according to claim 6, wherein
the control system further includes a display processing unit configured to display, on a display unit, at least one of a graph indicating a relationship between the circulation stability and elapsed time, a numerical value indicating the circulation stability, a graph indicating a relationship between the heart function stability and elapsed time, a numerical value indicating the heart function stability, a graph indicating a relationship between the comprehensive stability and elapsed time, and a numerical value indicating the comprehensive stability.
